# EUROPEAN PATENT SPECIFICATION

(11) **EP 4 167 957 B1**
(45) Date of publication and mention of the grant of the patent: **18.12.2024**
(21) Application number: 21733136.2
(22) Date of filing: 18.06.2021
(51) Int. Cl.: A61K 9/00, A61K 39/395, A61K 47/10, A61K 47/32, A61K 47/34

(54) **BIODEGRADABLE COMPOSITIONS AND IMPLANTS**
BIOLOGISCH ABBAUBARE ZUSAMMENSETZUNGEN UND IMPLANTATE
COMPOSITIONS ET IMPLANTS BIODÉGRADABLES

(30) Priority: 19.06.2020 EP 20181231
(43) Date of publication of application: 26.04.2023
(73) Proprietor: Re-Vana Therapeutics Ltd, Belfast BT7 1NF (GB)
(72) Inventor: THAKUR, Raghu Raj Singh, Belfast BT17 9HA (GB); JONES, David, Glengormley Newtownabbey BT365ZA (GB); SONAWANE, Rahul, Belfast BT9 7BL (GB); WANG, Yujing, Belfast BT7 1PT (GB)
(74) Representative: Gill Jennings & Every LLP
(86) International application number: PCT/EP2021/066670
(87) International publication number: WO 2021/255264

(56) References cited:
- WO-A1-2012/120139
- WO-A1-2017/081154
- CHRISTINE HIEMSTRA ET AL: "Rapidly in Situ Forming Biodegradable Robust Hydrogels by Combining Stereocomplexation and Photopolymerization", JOURNAL OF THE AMERICAN CHEMICAL SOCIETY, vol. 129, no. 32, 1 August 2007 (2007-08-01), US, pages 9918 - 9926, XP055293638, ISSN: 0002-7863, DOI: 10.1021/ja072113p

## Description

### FIELD OF THE INVENTION

The present invention relates to biodegradable compositions and implants for the controlled release of therapeutic agents. More particularly, the present invention relates to biodegradable ocular compositions and implants for the controlled release of therapeutic agents in the eye.

### BACKGROUND OF THE INVENTION

Chronic retinal diseases are the leading contributor to visual impairment and blindness worldwide. Loss of sight has a major personal impact on people's daily lives and has a profound economic impact on individuals, families, public health and society. The World Health Organization estimates that globally about 285 million people are visually impaired, of which 39 million are blind and 246 million have low vision. Diseases that originate in the posterior segment (PS) or back of the eye lead to permanent loss of vision if left untreated and account for most of the blindness, such as in age-related macular degeneration (AMD), diabetic retinopathy (DR), diabetic macular edema (DME), cytomegalovirus (CMV) retinitis, retinitis pigmentosa, uveitis and glaucoma. The PS of the eye, which includes the retina, choroid, and vitreous body, is difficult to access due to the recessed location within the orbital cavity. Therefore, delivery of therapeutic agents to the PS of the eye has remained one of the most challenging tasks for pharmaceutical scientists and retina specialists.

Multiple approaches have been used to deliver therapeutic agents to the PS of the eye such as systemic, topical, periocular (or transscleral) and intravitreal. Topical (e.g. eye drops) and systemic (e.g. oral tablets) routes result in low or sub-therapeutic agent levels due to multiple ocular barriers, requiring administration of unnecessarily high concentrations of therapeutic agent that causes therapeutic agent-related toxicity and producing low treatment efficacy.

WO2017081154A1 discloses ocular compositions that can be administered to the eye in various forms to achieve controlled release of a therapeutic agent. These compositions can be used to form ocular implants by crosslinking the formulation either *in situ* after injecting it into the eye of a patient or can be pre-formed prior to injecting in the eye.

There is a need for alternative systems for ocular delivery of therapeutic agents.

### SUMMARY OF THE INVENTION

In a first aspect, the invention relates to an ocular composition that can be administered to the eye in various forms to achieve controlled release of a therapeutic agent Such ocular composition comprises:
a) at least 0.1% w/w of a therapeutic agent;
b) 5 to 95% w/w of a photopolymerizable composition comprising 3 to 70% w/w of one or more compounds of formula I: 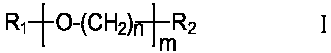 wherein R₁ is hydrogen or a linear or branched C₁-C₃ alkyl; R₂ is an acrylate or methacrylate group; n is 2 or 3 and m is equal or greater than 1, the weight percentage of the one or more compounds of formula I being based on the total weight of the photopolymerizable composition,
wherein the photopolymerizable composition further comprises one or more disubstituted acrylate or methacrylate compounds selected from the group consisting of ethylene glycol diacrylate, di (ethylene glycol) diacrylate, poly (ethylene glycol) diacrylate, ethylene glycol dimethacrylate, di (ethylene glycol) dimethacrylate, poly (ethylene glycol) dimethacrylate, propylene glycol diacrylate, di (propylene glycol) diacrylate, poly (propylene glycol) diacrylate, propylene glycol dimethacrylate, di (propylene glycol) dimethacrylate, poly (propylene glycol) dimethacrylate, 1,6-hexanediol dimethacrylate;
c) 0.1 to 40% w/w of a biodegradable polymer selected from the group consisting of lactide/glycolide copolymer (including poly(lactide-co-glycolide) (PLGA)), poly (L-lactide) (PLA), polyhydroxyalkanoates, including polyhydroxybutyrate, polyglycolic acid (PGA), polycaprolactone (PCL), poly (DL-lactide) (PDL), poly (D-lactide), lactide/caprolactone copolymer, poly-L-lactide-co-caprolactone (PLC) and mixtures, copolymers, and block copolymers thereof, and
d) a photoinitiator.

In a further aspect, the invention relates to a method of making the above ocular composition, implants based on the above ocular composition as well as ocular implants obtainable by such method.

In a still further aspect, the present invention relates to an ocular implant comprising at least 0.1% w/w of a therapeutic agent, 5 to 95% w/w of a crosslinked polymer matrix and 0.1 to 40% w/w of a biodegradable polymer as defined above, characterized in that the crosslinked polymer matrix is obtained by crosslinking a photopolymerizable composition as defined above.

The present invention provides ocular compositions and implants that can be administered to the eye in various forms to achieve controlled release of a therapeutic agent The invention allows the flexibility to administer a range of small and large therapeutic molecules, including proteins, peptides and gene therapeutics, and to maintain their activity for a controlled period of time.

The presence of one or more compounds of formula I in the photopolymerizable composition enables to optimize delivery and complete degradation of the implant after ceasing to release the therapeutic compound.

The degradation of the implants according to the present invention occurs along with the drug release. Complete degradation of the exhausted implant also enables the injection of additional implants in case the therapeutic treatment must be continued. Even if the organ may simultaneously host more than one implant, the coexistence time of the old and new implant should be in any case minimized.

The compositions and implants of the present invention show delivery profiles which can release therapeutically effective amounts of drug over extended periods of time while fully degrading after completion of drug release.

Furthermore, the implants according to the present invention maintain the physical structure during degradation and, accordingly do not disintegrate into a plurality of particles which could cause, among others, a series of unwished mini burst effects within the organ. The formation of particle debris in the front and/or the back of the eye, with the potential risk to clog the trabecular meshwork, is therefore avoided and proper drainage of the aqueous humors from the eye is maintained.

### DESCRIPTION OF THE FIGURES:

Fig. 1 shows the *in vitro* accelerated degradation profile of O1, 02, 03 and 04 implants in 10mM NaOH at 37°C at a shaking speed of 40 rpm.
Fig. 2 shows the daily OVA release per implant (µg) profile (Y1 axis) vs. % implant weight (Y2 axis) of O1, O2, O3 and 04 implants in PBS (pH 7.4) at 37°C and shaking speed of 40 rpm.
Fig. 3 shows the Scanning Electronic Microscopy (SEM) images of morphological changes during ambient condition degradation of compositions O1 and O4 implants in 10 mM PBS (pH 7.4) at 37°C and shaking speed of 40 rpm.
Fig. 4 shows the *in vitro* accelerated degradation profile of OV 1, OV 2, OV 3 and OV 4 in 10mM NaOH at 37°C at a shaking speed of 40 rpm.
Fig. 5 shows the *in vitro* accelerated degradation profile of OV 2 and OV 6 in 10mM NaOH at 37°C at a shaking speed of 40 rpm.
Fig. 6 shows the daily OVA release per implant (µg) profile (Y1 axis) vs. % implant weight (Y2 axis) of OV 1, OV 2, OV 3, OV 4 and OV 6 implants in PBS (pH 7.4) at 37°C and shaking speed of 40 rpm.
Fig. 7 shows the accelerated degradation profile of DEX 1, DEX 2 and DEX 3 implants in 50 mM NaOH at 37°C and shaking speed of 40 rpm.
Fig. 8 shows the daily DEX release per implant (µg) profile (Y1 axis) vs. % implant weight (Y2 axis) of DEX 1, DEX 2 and DEX 3 implants in PBS (pH 7.4) at 37°C and shaking speed of 40 rpm.
Fig. 9 shows the accelerated degradation profile of LP 1, LP 2 and LP 3 implants in 50 mM NaOH at 37°C and shaking speed of 40 rpm.
Fig. 10 shows the daily LP release per implant (µg/mL) profile (Y1 axis) vs. implant weight (Y2 axis) of LP 1, LP 2 and LP 3 implants in PBS (pH 7.4) at 37°C and shaking speed of 40 rpm.
Fig.11 shows the accelerated degradation profile of F19, B6 and B7 implants in 50 mM NaOH and incubated in static incubator at 37°C.
Fig. 12 shows the daily BEZ release per implant (µg/mL) profile in PBS (pH 7.4) (Y1 axis) vs. percentage implant weight (Y2 axis) in 10 mM NaOH of F19, B6 and B7 implants incubated in static incubator at 37°C.
Fig. 13 shows the *in vitro* accelerated degradation profile of B1, B2 and B3 implants in 10mM NaOH at 37°C at a shaking speed of 40 rpm.
Fig. 14 shows the daily OVA release per implant (µg) profile (Y1 axis) vs. % implant weight (Y2 axis) of B1, B2 and B3 implants in PBS (pH 7.4) at 37°C and shaking speed of 40 rpm.

### DETAILED DESCRIPTION OF THE INVENTION

As used herein, and unless specified otherwise, the term "% w/w" means the weight percentage of a given component over the total weight of the copolymer, the composition or the implant including such component, as the case may be.

As used herein, "biodegradable" is the chemical degradation by biological means. In some embodiments, the biodegradation is 100%, 98%, 90%, 85%, 80%, 60%, 50%, or 45% degradation of one or more of the compositions, monomers, oligomers, fragments, polymers, photoinitiators, solvents, co-solvents, or co-initiators.

As used herein "copolymer" is a mixture of two or more different types of monomer units. As used herein "block copolymer" is a mixture of two or more homopolymer subunits.

The therapeutic agent of the present invention can be selected from a wide range of small and large molecules. Exemplary therapeutic agents include, but are not limited to, polypeptides, nucleic acids, such as DNA, RNA, and siRNA, growth factors, steroid agents, antibody therapies, including bispecific antibodies, antimicrobial agents, antibiotics, antiretroviral therapeutic agents, anti-inflammatory compounds, antitumor agents, anti-angiogeneic agents, anti-VEGF (Vascular Endothelial Growth Factor) agents, chemotherapeutic agents, miscellaneous ophthalmic agents, mydriatic agents, ophthalmic anesthetics, ophthalmic anti-infectives, ophthalmic anti-inflammatory agents, ophthalmic antihistamines and decongestants, ophthalmic diagnostic agents, ophthalmic glaucoma agents, ophthalmic lubricants and irrigations, ophthalmic steroids, ophthalmic steroids with anti-infectives, ophthalmic surgical agents, tyrosine kinase inhibitors.

In some other embodiments, the molecular weight of the therapeutic agent is greater than 200 Da, 500 Da, 1000 Da, 10 kDa, 30 kDa, 50 kDa, 75 kDa, 100 kDa, 150 kDa, 200 kDa and 250 kDa.

In one embodiment, the therapeutic agent of the present invention includes, but is not limited to, ketorolac, naphazoline, lidocaine, bevacizumab, aflibercept, brolucizumab, pegaptanib, brimonidine tartrate, dorzolamide, bromfenac sodium, azithromycin, rapamycin, bepotastine besilate, diclofenac, besifloxacin, cysteamine hydrochloride, fluocinolone acetonide, difluprednate, tasimelteon, ocriplasmin, enoxaparin sodium, ranibizumab, latanoprost, timolol maleate, bimatoprost, ofloxacin, cephazolin, phenylephrine, dexamethasone, triamcinolone acetonide, levofloxacin, cyclophosphamide, melphalan cyclosporine, methotrexate, azathioprine, travoprost, verteporfin, tafluprost, ketotifen fumarate, foscarnet, amphotericin B, fluconazole, voriconazole, ganciclovir, acyclovir, gatifloxacin, mitomycin-C, prednisolone, prednisone, vitamin (vitamin A, vitamin C, and vitamin E), zinc, copper, lutein, zeaxanthin or combinations thereof.

In another embodiment, the therapeutic agent of the present invention is dexamethasone, timolol maleate, brimonidine tartrate, triamcinolone acetonide, bromfenac sodium, latanoprost or mixtures thereof.

In one embodiment, the compositions or implants of the present invention can deliver bioactive agents, a large molecular weight therapeutic agent, such as, aflibercept, pegaptanib, or an antibody therapeutic, such as ranibizumab, bevacizumab, trastuzumab, rituximab, gentuzumab, ozagamicin, brolucizumab, cetuximab, faricimab, conbercept or biosimilars thereof.

In one embodiment, the therapeutic agent of the present invention is ranibizumab, bevacizumab, latanoprost, dexamethasone or timolol maleate.

According to other embodiments of the present invention, the therapeutic agent is present in an amount between 0.5 and 70% w/w, 5 and 70% w/w, 10 and 70% w/w, 20 and 70% w/w, 30 and 70% w/w, 40 and 70% w/w, 50 and 70% w/w, 5 and 50% w/w, 10 and 50% w/w, 20 and 50% w/w, 30 and 50% w/w, and between 40 and 50% w/w of the total weight of the ocular composition or ocular implant

The therapeutic agent can be used as such or in form of a solution wherein an amount of therapeutic agent is dissolved in a suitable solvent The therapeutic agent can also be freeze-dried or spray-dried before being used in the preparation of the ocular composition of the present invention in order to facilitate the incorporation of high concentrations of the therapeutic agent into the implant The amount of the therapeutic agent to be dissolved depends on the final loading that the ocular composition or implant has to have. The choice of the solvent depends on the polarity of the therapeutic agent.

According to an embodiment of the present invention, the solvent can be selected from water, dimethyl sulfoxide, decylmethyl sulfoxide, 2-pyrrolidone, 1-methyl-2-pyrrolidone, N-vinyl-pyrrolidine, N-Methyl-2- pyrrolidone, N-ethyl-pyrrolidone, glycerol formal, glycerol, polyethylene glycol, propylene glycol, benzyl alcohol, benzyl benzoate, ethyl benzoate, triacetin, triethyl citrate, dimethylformamide, dimethylacetamide and tetrahydrofuran.

In one embodiment, co-solvents may be used, and they can be selected from dichloromethane, tetrahydrofuran, ethyl acetate, acetone, dimethylformamide, acetonitrile, acetic acid, methanol, ethanol, isopropanol, glycofurol or butanol.

In case of hydrophilic therapeutic agents, the solvent may be an aqueous based solvent such as water or a phosphate buffered saline (PBS) solution.

According to another embodiment, the solvent may be selected from dimethyl sulfoxide, decyl methyl sulfoxide, 2-pyrrolidone, 1-methyl-2-pyrrolidne, N-methyl-2-pyrrolidone and glycerol formal.

Furthermore, the above-described solvents and co-solvents can be used in the preparation of any of the compositions and implants of the invention, in combination with any of the other photopolymerizable compositions, biodegradable polymers, photoinitiators, pore forming agents, and co-initiators described herein.

The photopolymerizable fragments or monomers of the present invention can be used in any of the compositions and implants of the invention in combination with any of the other biodegradable polymers, therapeutic agents, photoinitiators, solvents, co-solvents, drug modulating agents and co-initiators described herein or known in the common general knowledge.

In one embodiment, complete biodegradation takes place over a period between one and four times the total drug release time of the implant As an example of this embodiment, an implant of the invention delivering drug during a period of three months should completely degrade within between three and twelve months from injection into the organ.

As used herein, the term "photopolymerizable composition" is a composition which can form a crosslinked polymer network upon exposure to light, in particular UV light As used herein, photopolymerizable compositions include photopolymerizable monomers and oligomers (such as, dimers, trimers, and tetramers). The terms "oligomers" and "fragments" can be used interchangeably to mean between two and twenty monomers, optionally between two and ten monomers, further optionally between two and five monomers or between two and four monomers. A "photopolymerizable monomer" is a single unit of a photopolymerizable polymer that can bind chemically to other monomers to form a polymer.

Photopolymerizable compositions of the present invention can be crosslinked with UV radiation to form the crosslinked polymer matrix of the ocular implant of the present invention.

In one embodiment, R₁ of the one or more compounds of formula I are independently selected from hydrogen and methyl. In still another embodiment, n in formula I is equal to 2.

According to a further embodiment of the present invention, the compound of formula I is selected from the group consisting of poly(ethylene glycol) acrylate, poly(ethylene glycol) methacrylate, poly(ethylene glycol) methyl ether acrylate, poly(ethylene glycol) methyl ether methacrylate, ethylene glycol methyl ether acrylate, di(ethylene glycol) ethyl ether acrylate, ethylene glycol methyl ether methacrylate, di(ethylene glycol) ethyl ether methacrylate and mixtures thereof. In one embodiment, the one or more compound of formula I is poly (ethylene glycol) methacrylate (PEGMA).

In one embodiment of the present invention, the photopolymerizable composition comprises 5 to 60% w/w, 5 to 45% w/w or 5 to 20% w/w or 10 to 15% w/w of the one or more compounds of formula I, the weight percentage being based on the total weight of the photopolymerizable composition.

In another embodiment, the photopolymerizable composition further comprises one or more disubstituted acrylate or methacrylate compounds selected from the group consisting of ethylene glycol diacrylate, di (ethylene glycol) diacrylate, poly (ethylene glycol) diacrylate, ethylene glycol dimethacrylate, di (ethylene glycol) dimethacrylate, poly (ethylene glycol) dimethacrylate, propylene glycol diacrylate, di (propylene glycol) diacrylate, poly (propylene glycol) diacrylate, propylene glycol dimethacrylate, di (propylene glycol) dimethacrylate, poly (propylene glycol) dimethacrylate, 1,6-hexanediol dimethacrylate.

In one embodiment of the present invention, the weight ratio between such one or more disubstituted acrylate or methacrylate compounds and the one or more compounds of formula I is between 0.5 and 20, between 0.5 and 15, between 1 and 15, between 1 and 10 and between 2 and 5.

In another embodiment, the one or more disubstituted acrylate or methacrylate compounds in the photopolymerizable composition is polyethylene glycol diacrylate (PEGDA) or polyethylene glycol dimethacrylate (PEGDMA).

In yet another embodiment, the one or more disubstituted acrylate or methacrylate compounds in the photopolymerizable composition is polyethylene glycol diacrylate (PEGDA).

In yet another embodiment, the photopolymerizable composition further comprises polyethylene glycol diacrylate (PEGDA).

In yet another embodiment, the weight ratio PEGDA:PEGMA is between 0.5 and 20, between 0.5 and 15, between 1 and 15, between 1 and 10 and between 2 and 5.

PEGMA and PEGDA are synthetic polymers, available in different molecular weights. They are extremely amenable to mechanical, structural and chemical alteration and so resulting in hydrogels with variable properties in terms of drug delivery and other biomedical applications. PEGMA and PEGDA are formed through the functionalization of one or both ends, respectively, of each PEG molecule with an acrylate group. PEGMA and PEGDA are non-toxic, eliciting only a minimal immunogenic response. PEGMA and PEGDA have double-bond containing acrylate end groups which show rapid polymerization when exposed to light in the presence of an appropriate initiator to produce a hydrogel network.

The average molecular weight of the photopolymerizable compositions of the present invention is typically between 100 and 300,000 Da, between 200 to 100,000 Da, between 200 to 50,000 Da, between 200 to 20,000 Da, between 200 to 10,000 Da, between 200 and 8,000 Da, between 200 and 5,000 Da, or between 200 and 1,000 Da.

The photopolymerizable compositions of the present invention typically have viscosities between 0.1 to 7 dL/g, between 0.2 to 5 dL/g, or between 0.5 to 2 dL/g.

In an embodiment, the photopolymerizable composition is present in an amount between 10 and 75% w/w, between 20 and 75% w/w, between 30 and 75% w/w, between 40 and 75% w/w, between 45 and 75% w/w, of the total weight of the ocular composition.

The biodegradable polymers of the present invention can be used in any of the compositions and implants of the invention in combination with any of the other photopolymerizable compositions, therapeutic agents, photoinitiators, solvents, co-solvents, therapeutic agent release modulating agents and co-initiators described herein or known in the common general knowledge.

The biodegradable polymers of the present invention are biodegradable but not photopolymerizable.

In another embodiment, the biodegradable polymer is selected from the group consisting of lactide/glycolide copolymer (including poly(lactide-co-glycolide) (PLGA)), poly (L-lactide) (PLA), polyhydroxyalkanoates, including polyhydroxybutyrate, polyglycolic acid (PGA), polycaprolactone (PCL), poly (DL-lactide) (PDL), poly (D-lactide), lactide/caprolactone copolymer, poly-L-lactide-co-caprolactone (PLC) and mixtures, copolymers, and block copolymers thereof.

In one embodiment, the biodegradable polymer is lactide/glycolide copolymer (including poly(L-lactide-co-glycolide) (PLGA)), poly (L-lactide) (PLA), poly(DL-lactide) (PDL), and lactide/caprolactone copolymer (PLC).

In a particular embodiment, the biodegradable polymer is poly(lactide-co-glycolide) (PLGA).

PLGA is typically prepared by polymerization of lactic acid and glycolic acid monomers. The glass transition temperatures (Tg) of PLGA copolymers are above physiological temperatures of 37 °C, which imparts a moderately rigid chain configuration and therefore the mechanical strength at ambient temperatures. The use of PLGA in different lactide (LA) to glycolide (GA) ratio and molecular weights allows different drug release profiles. In one embodiment, the molar ratio of lactic acid to glycolic acid in the PLGA is 90% lactic acid to 10% glycolic acid, 85% lactic acid to 15% glycolic acid, 75% lactic acid to 25% glycolic acid, 65% lactic acid to 35% glycolic acid, 50% lactic acid to 50% glycolic acid, 35% lactic acid to 65% glycolic acid, 25% lactic acid to 75% glycolic acid, 15% lactic acid to 85% glycolic acid, and 10% lactic acid to 90% glycolic acid.

In another embodiment, the biodegradable polymer is PCL, PLC, PLA, or mixtures, copolymers, or block copolymers thereof.

In an embodiment, the biodegradable polymer is present in an amount between 1 and 40% w/w, between 1 and 30% w/w, between 1 and 20% w/w, between 5 and 20% w/w, between 2 and 10% w/w, between 5 and 10% w/w, between 1 and 5% w/w of the total weight of the ocular composition.

The photoinitiators described herein can be used in any of the compositions and implants of the present invention in combination with any of the other photopolymerizable compositions, biodegradable polymers, therapeutic agents, photoinitiators, solvents, co-solvents, and co-initiators described herein.

In certain embodiments, the photoinitiator is designed to work using light from 200 to 550 nm. In some embodiments, a photoinitiator is designed to work using UV light from 200 to 500 nm. In other embodiments, a photoinitiator is designed to work using UV light from 200 to 425 nm.

In certain embodiments, the light source may allow variation of the wavelength of light and/or the intensity of the light. Light sources useful in the present invention include, but are not limited to, laser diodes and lamps. Fiber optic devices can be used to transmit the light.

In another embodiment, the photoinitiator may be selected from a hydroxyketone photoinitiator, an amino ketone photoinitiator, a hydroxy ketone/benzophenone photoinitiator, a benzyldimethyl ketal photoinitiator, a phenylglyoxylate photoinitiator, an acyl phosphine oxide photoinitiator, an acyl phosphine oxide/alpha hydroxy ketone photoinitiator, a benzophenone photoinitiator, a ribityl isoalloxazine photoinitiator, a peroxide photoinitiator, a persulfate photoinitiator or a phenylglyoxylate photoinitiator or any combination thereof. Optionally, the photoinitiator is 2-Hydroxy-4'-(2-hydroxyethoxy)-2-methylpropiophenone, 1-[4-(2- hydroxyethoxy)-phenyl]-2-hydroxy-2-methyl-1- propanone, 2,2-dimethoxy-2-phenylacetophenone (DMPA), diphenyl(2,4,6-trimethylbenzoyl) phosphine oxide (DPPO) or riboflavin. In another embodiment, the photoinitiator is benzoyl peroxide, 2,2"-azobisisobutyronitrile, dicumyl peroxide, lauroyl peroxide and/or camphorquinone.

In one embodiment the photoinitiator is 1-[4-(2-hydroxyethoxy)-phenyl]-2-hydroxy-2- methyl-1-propanone (Irgacure 2959), phenyl-bis(2,4,6-trimethylbenzoyl)- phosphineoxide (Irgacure 819) and mixtures thereof.

In one embodiment, the photoinitiator is present in an amount varying between 0.1 and 5% w/w of the total weight of the ocular composition.

In one embodiment, the compositions of the present invention further comprise a co-initiator. In one embodiment, the co-initiator is triethanolamine, dimethylaminobenzoate (DMAB), trimethylolpropane, L-arginine.

In another embodiment, the photoinitiator is riboflavin and the co-initiator is L-arginine.

According to another embodiment of the present invention, the ocular composition comprises or consists of:
a) 10 to 50% w/w of the therapeutic agent
b) 1 to 20% w/w of poly(lactide-co-glycolide) (PLGA)
c) 30 to 88.99% w/w of the photopolymerizable composition consisting of poly(ethylene glycol) methacrylate (PEGMA) and poly (ethylene glycol) diacrylate (PEGDA).
d) 0.01 to 5% w/w of a photoinitiator selected from the group consisting of 1-[4-(2-hydroxyethoxy)-phenyl]-2-hydroxy-2- methyl-1-propanone, phenyl-bis(2,4,6-trimethylbenzoyl)- phosphineoxide and mixtures thereof.
wherein the weight ratio PEGDA:PEGMA is between 0.5 and 20, between 0.5 and 15, between 1 and 15, between 1 and 10 and between 2 and 5.

According to still another embodiment of the present invention, the ocular composition comprises or consists of:
a) 20 to 50% w/w of the therapeutic agent
b) 5 to 20% w/w of poly(lactide-co-glycolide) (PLGA)
c) 30 to 74.99% w/w of the photopolymerizable composition consisting of poly(ethylene glycol) methacrylate (PEGMA) and poly (ethylene glycol) diacrylate (PEGDA).
d) 0.01 to 5% w/w of a photoinitiator selected from the group consisting of 1-[4-(2-hydroxyethoxy)-phenyl]-2-hydroxy-2- methyl-1-propanone, phenyl-bis(2,4,6-trimethylbenzoyl)- phosphineoxide and mixtures thereof.
wherein the weight ratio PEGDA:PEGMA is between 0.5 and 20, 0.5 and 15, 1 and 15, 1 and 10 or 2 and 5.

In one embodiment, the therapeutic agent is ranibizumab, bevacizumab, latanoprost, dexamethasone or timolol maleate.

In one embodiment, the composition of the present invention comprises a release modulating agent A suitable release modulating agent may be selected in view of the specific therapeutic agent and composition of the implant, as well as the desired elution profile or release rate. The release modulating agent may be a naturally occurring agent or polymer or a synthetic agent or polymer.

All release modulating agents described herein can be used in any of the implants and compositions of the invention in combination with any of the other photopolymerizable compositions, biodegradable polymers, therapeutic agents, photoinitiators, solvents, co-solvents, and co-initiators described herein.

The release modulating agents may be present in amounts between 0.1 and 40% w/w, between 1 and 30% w/w, between 1 and 20% w/w, between 1 and 10% w/w, between 5 and 10% w/w.

Optionally, the release modulating agent alters water absorption into the implant matrix, thus controlling the release rate of the therapeutic agents and the implant degradation. In an embodiment, a suitable water absorption modulating agent is one or more polysaccharides like for example chitosan and cellulose based materials including hydroxypropyl methylcellulose (HPMC); hyaluronic acid; poloxamer; polyether like for example polyethylene glycol; gelatin; polyvinylpyrrolidone; polyvinyl alcohol and mixtures thereof. In one embodiment, suitable water absorption modulating agents are hydroxypropyl methylcellulose (HPMC) and polyethylene glycol (PEG).

In one embodiment, the release modulating agent is a pore-forming agent and/or a stability enhancing agent. Optionally, it is lactose, maltose, glucose, mannitol, sodium chloride, magnesium carbonate, magnesium hydroxide, potassium chloride, sodium bicarbonate, ammonium bicarbonate, potassium bicarbonate, agarose or sucrose.

In another embodiment, the release modulating agent is a mixture of two or more modulating agents described above, in order to provide more than one functionality to the ocular composition or implant of the present invention. Optionally, the release modulating agent is polyethylene glycol, hydroxypropyl methylcellulose (HPMC) or mixtures thereof.

Optionally, implant porosity can be adjusted by preparing implants in the presence of dispersed water-soluble porosinogens, which can be removed later by washing with water to leave an interconnected meshwork (i.e., porous hydrogels). The pore size of hydrogels prepared by the porosinogen technique depends on the size of the porosinogens.

In another embodiment of the present invention, the ocular composition does not contain any release modulating agent.

Another aspect of the present invention is to provide an ocular composition as described above, for use in the preparation of an ocular implant. Alternatively, the ocular composition of the present invention is used to coat an ocular implant or a support for an ocular implant.

A further aspect of the present invention is a method of making an ocular composition as described above. The method comprises a step of dissolving the therapeutic agent into a solvent/co-solvent to obtain a solution or suspension and, subsequently, a step of mixing, in any order of addition, the so obtained therapeutic agent solution with the polymerizable composition, the biodegradable polymer, the photoinitiator and optionally the release modulating agent Optionally, the therapeutic agent is first mixed with the photopolymerizable composition and the so obtained mixture is mixed, in any order of addition, with the biodegradable polymer, the photoinitiator and optionally the release modulating agent Alternatively, the therapeutic agent is first mixed with a portion of the photopolymerizable composition and another portion of photopolymerizable composition is mixed with the biodegradable polymer, the photoinitiator and optionally the release modulating agent

The choice of the solvent which can be used according to the present invention depends on the polarity of the therapeutic agent.

Optionally, the solvent can be selected from water, dimethyl sulfoxide, decylmethyl sulfoxide, 2-pyrrolidone, 1-methyl-2-pyrrolidne, N-vinyl-pyrrolidine, N-Methyl-2-pyrrolidone, N-ethyl-pyrrolidone, glycerol formal, glycerol, polyethylene glycol, propylene glycol, benzyl alcohol, benzyl benzoate, ethyl benzoate, triacetin, triethyl citrate, dimethylformamide, dimethylacetamide, acetonitrile, dicloromethane and tetrahydrofuran.

In one embodiment, co-solvents may be used, and they can be selected from dichloromethane, tetrahydrofuran, ethyl acetate, acetone, dimethylformamide, acetonitrile, acetic acid, methanol, ethanol, isopropanol, glycofurol or butanol.

In case of hydrophilic therapeutic agents, the solvent may be an aqueous based solvent such as water or phosphate buffered saline (PBS) solution.

According to another embodiment, the solvent may be selected from dimethyl sulfoxide, decylmethyl sulfoxide, acetonitrile, 2-pyrrolidone, 1-methyl-2-pyrrolidne, N-methyl-2-pyrrolidone and glycerol formal.

Alternatively, the therapeutic agent is not dissolved into a solvent prior to mixing it with the other components. Accordingly, the therapeutic agent, the polymerizable composition, the biodegradable polymer, the photoinitiator and optionally the release modulating agent are mixed together in any order of addition. Alternatively, the therapeutic agent is first mixed with a portion of the photopolymerizable composition and another portion of photopolymerizable composition is mixed with the biodegradable polymer, the photoinitiator and optionally the release controlling agent

A further aspect of the present invention is a method of making an ocular implant comprising a step of providing an ocular composition of the present invention and, subsequently, a step of irradiating the ocular composition with light at a wavelength between 200 and 550 nm, between 200 and 500 nm, between 200 and 490 nm, or between 200 to 425 nm, for a period of time between 1 second and 60 minutes, between 30 seconds and 30 minutes, between 2.5 minutes and 20 minutes, between 5 minutes and 10 minutes. In one embodiment, the crosslinking is for 3 seconds, 6 seconds, 9 seconds, 15 seconds, 30 seconds, 1, 2.5, 5, 10, 20 or 30 minutes.

Not claimed, but disclosed is an ocular implant obtainable by the method mentioned above.

In one embodiment, polymer molecular weight, type and copolymer ratio, drug type and loading, implant size, time and extent of UV crosslinking, amount and type of photoinitiator, release modulating agent, solvent and/or co-solvent can be altered to control the rate and extent of drug release. The alteration of these factors provides compositions of the invention that can be easily tailored to produce desired period of drug release to address specific clinical/patient needs in treating various ocular diseases.

The ocular compositions of the invention can be:
i) injected into the eye followed by application of short-term UV light to induce in situ photocrosslinking, resulting in implant formation, termed as In Situ Photocrosslinked Implants (ISPcI); and
ii) photocrosslinked prior to application in the eye to form an implant of desired shape and size (e.g. film, rod or nano/microparticles) that can be administered intraocularly to provide desired period of drug delivery, termed as Preformed Photocrosslinked Implants (PPcI).

Alternatively, the compositions of the invention can be used to coat ocular devices, including both in situ and pre-formed ocular devices.

The implants of the present invention can be of any desired shape such as but not limited to, rectangular, square, spherical, cylindrical, circular, oval, films, dumbbell, rods and beads.

The implants of the present invention can have any desired size and can be, for example, in the macro, micro or nano particle size range.

In one embodiment of the present invention, the ocular implant is an implant which is less than 10 mm or less than 5 mm or less than 3 mm in one of the dimensions. In one embodiment, the implant is a rectangular implant of dimensions 10 × 5 × 0.5 mm. In one embodiment, the implant (ISPcI) is a spherical implant of diameter less than 10 mm or less than 5 mm or less than 3 mm. In one embodiment of the present invention, the ocular implant is a nanoparticle or a microparticle.

In one embodiment, the nanoparticle ocular implant is less than 1,000 nm, less than 900 nm, less than 750 nm, less than 500 nm, or less than 100 nm.

In one embodiment, the microparticle ocular implant is less than 1,000 µm, less than 900 µm, less than 750 µm, less than 500 µm, or less than 25 µm.

Optionally, the implant is an in situ formed ocular implant. Alternatively, the implant is a pre-formed ocular implant.

In Situ Photocrosslinked Implants (ISPcI) according to the present invention are those that form and take up their final localized structure once they are inserted into the body. The ability of the ISPcI to fill irregular defects is one of their advantage. The ISPcIs of the present invention also have additional advantages, which include, site-specific action due to relatively easy and less invasive application, localized delivery to specific tissues, prolonged delivery times, reduction in side effects linked with systemic delivery and also superior patient comfort and compliance. An additional advantage of the ISPcIs of the present invention is that it does not require extreme pH conditions or elevated temperatures during processing, which could cause issues when working with temperature or pH labile drugs like proteins, peptides or genetic material. Furthermore, rapid crosslinking at physiological temperatures can swiftly entrap drug molecules and can result in an ISPcI that provides long-term controlled drug release. Photocrosslinking is also beneficial in comparison to spontaneous crosslinking (e.g. enzymatic, self-assembled, Michael addition) as the initiation of the process is only triggered when exposed to a light source, therefore premature gelation is not an issue resulting in excellent control of material formation. Furthermore, short-term application of UV light will not cause any safety issues as it is considered safe for ocular applications, as UV light is clinically used for corneal crosslinking. Importantly, administration by this method allows the injection of a relatively low viscosity material into the body, which then solidifies due to phase inversion and forms a semi-solid depot that upon photocrosslinking controls the drug delivery to provide short or long-term therapeutic action.

In one embodiment, the ISPcIs are formed by injection of an ocular composition of the present invention into a subject in need thereof and subsequent crosslinking using external source of UV light that results in formation of a solid implant which controls drug release for a desired period of time.

For ISPcIs of the invention the molecular weight of the photopolymerizable composition is typically between 100 and 6,000 Da, between 200 and 3,000 Da, or between 200 and 1,000 Da.

Preformed Photocrosslinked Implants (PPcI) of the present invention can be inserted in the eye, for example in the fornix, subconjunctively, intracameral, intrastromal/intracorneal, transsclerally/periocular, intrasclerally or intravitreally, subretinal, to treat the front of the eye or back of the eye diseases. The PPcI can be fabricated in a variety of shapes including, but not limited to, rods, films, cylindrical or circular and sizes, including in the form of micro or nanoparticles.

In one embodiment, PPcI nano and microparticles are obtained by sonicating the mixture of therapeutic agent, photopolymerizable composition, biodegradable polymer, photoinitiator and, optionally, release modulating agent in an aqueous medium. In one embodiment, the aqueous medium is a combination of water and phosphate buffered saline (PBS). Irradiation can be applied during sonication i.e. sonicating the mixture under UV light or it can alternatively occur after the sonification step.

The PPcIs of the present invention have the advantage of high crosslink density and/or a tight polymer network structure which can be configured to control drug release and/or eliminate any burst release.

The PPcIs of the present invention can be fabricated to have a single and/or multiple layer which will enable loading of more than one drug or the same drug with different release profiles or rates.

Furthermore, the rate of degradation of the implants can be slower for the PPcIs than for the ISPcIs of the invention and can be controlled in function of the specific diseases or disorders to be treated.

For the PPcIs of the invention the average molecular weight of the photopolymerizable polymers is typically between 100 and 300,000 Da, between 200 to 100,000 Da, between 200 to 50,000 Da, between 200 to 20,000 Da, or between 200 to 10,000 Da.

In one embodiment, the present invention is a PLGA/PEGDA/PEGMA PPcI. In another embodiment, the present invention is a PLGA/PEGDA/PEGMA ISPcI.

In one embodiment, the polymers of the photopolymerizable composition, like for example PEGDA and PEGMA, can act as a solvent before they are crosslinked *in situ* to form an ISPcI. The polymers of the photopolymerizable composition also help to prevent dispersion of the different components of the overall composition within the organ after injection and before they are immobilized upon crosslinking.

In one embodiment, the biodegradable polymer is essentially contained within a matrix of the photopolymerizable composition. Optionally, the biodegradable polymer is essentially contained within a matrix of the photopolymerizable composition that forms a gel upon mixing. In one embodiment the photopolymerizable polymer is crosslinked in presence of a photoinitiator and the biodegradable polymer and therapeutic agent(s). In one embodiment, the biodegradable polymer is hydrophobic in nature and the photopolymerizable polymer is hydrophilic in nature. In one embodiment, the degree of crosslinking of the composite implant will govern the rate and extent of release of the therapeutic agent(s).

Another aspect of the present invention is an ocular implant comprising at least 0.1% w/w of a therapeutic agent, 5 to 95% w/w of a crosslinked polymer matrix and 0.1 to 40% w/w of a biodegradable polymer selected from the group consisting of lactide/glycolide copolymer (including poly(lactide-co-glycolide) (PLGA)), poly (L-lactide) (PLA), polyhydroxyalkanoates, including polyhydroxybutyrate, polyglycolic acid (PGA), polycaprolactone (PCL), poly (DL-lactide) (PDL), poly (D-lactide), lactide/caprolactone copolymer, poly-L-lactide-co-caprolactone (PLC) and mixtures, copolymers, and block copolymers thereof, characterized in that:
a) the crosslinked polymer matrix is obtained by crosslinking a photopolymerizable composition comprising 3 to 70% w/w of one or more compounds of formula I: 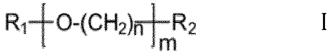 wherein R₁ is hydrogen or a linear or branched C₁-C₃ alkyl; R₂ is an acrylate or methacrylate group, n= 2 or 3 and m is equal or greater than 1, the weight percentage of the one or more compounds of formula I being based on the total weight of the photopolymerizable composition,
   wherein the photopolymerizable composition further comprises one or more disubstituted acrylate or methacrylate compounds selected from the group consisting of ethylene glycol diacrylate, di (ethylene glycol) diacrylate, poly (ethylene glycol) diacrylate, ethylene glycol dimethacrylate, di (ethylene glycol) dimethacrylate, poly (ethylene glycol) dimethacrylate, propylene glycol diacrylate, di (propylene glycol) diacrylate, poly (propylene glycol) diacrylate, propylene glycol dimethacrylate, di (propylene glycol) dimethacrylate, poly (propylene glycol) dimethacrylate, 1,6-hexanediol dimethacrylate;
b) the therapeutic agent and the biodegradable polymer are embedded in the polymer matrix.

As used herein, "embedded" means that the therapeutic agent is essentially trapped within the crosslinked polymer matrix and it is homogeneously dispersed or dissolved in the crosslinked polymer matrix and/or the biodegradable polymer.

In the compositions and implants of the present invention, varying the UV crosslinking time can control the rate of and duration of drug release. In some embodiments, an increase in UV crosslinking times causes a decrease in drug release. Additionally, varying the concentration of the photoinitiator can control the rate and duration of drug release. Furthermore, varying both the UV crosslinking time and the concentration of photoinitiator can control the rate and duration of drug release. In one embodiment, adding a pore-forming agent (e.g. MgCO₃), increases the drug release rate. In one embodiment, higher UV crosslinking time and higher concentration of photoinitiator can sustain the drug release for longer periods of time. In one embodiment, the drug release can be sustained for a period of greater than 1 day, 2 days, 1 week, 1 month, 2 months, 3 months, 6 months, 9 months, 12 months, 18 months, or 24 months.

In some embodiments, a controlled degradation rate of the ISPcIs and PPcIs of the present invention provide protection of the sensitive molecules such as peptides and proteins.

In some embodiments, burst release can be eliminated or controlled by varying the UV crosslinking time and varying formulation composition, implant volume.

In one embodiment, the present invention is a PPcI with no or low burst release. In one embodiment, the present invention is a PPcI with high crosslinking density that significantly slows drug diffusion.

Any of the implants and compositions described herein are suitable for use in any of the methods of the invention described herein.

Not claimed, but disclosed is a method of treating a disease or disorder of the eye in a subject in need thereof, comprising administering a composition or implant of the present invention to an ocular area of the subject

Not claimed, but disclosed is a composition or implant of the present invention for use in treating a disease or disorder of the eye in a subject in need thereof.

As used herein, an "ocular area" is an area inside, outside or adjacent to the eye of the subject In one embodiment, the ocular area is the sclera (intrascleral), outside the sclera (transscleral), the vitreous body, the choroid, the cornea, the stroma, intracameral, the aqueous humor, the lens, the fornix, or the optic nerve.

In one embodiment, the compositions and implants can be administered by injection, including, intravitreal, subconjunctival, peribulbar, subtenon or retrobulbar injections and onto the cornea.

In some embodiments, the implants are administered via a surgical procedure. In some embodiments, the implants are secured in place, after surgical implantation, via an adhesive or sutures.

The term "subject" refers to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In one embodiment, the subject is a non-human animal such as a farm animal (e.g., a horse, cow, pig or sheep), or a pet (e.g., a dog, cat, guinea pig or rabbit). In another embodiment, the subject is a "human".

As used herein, the terms "treat", "treatment" and "treating" refer to therapeutic treatments includes the reduction or amelioration of the progression, severity and/or duration of a disease, disorder or condition, or the amelioration of one or more symptoms (specifically, one or more discernible symptoms) of a disease, disorder or condition, resulting from the administration of the compositions or implant of the invention. In specific embodiments, the therapeutic treatment includes the amelioration of at least one measurable physical parameter of a disease, disorder or condition. In other embodiments the therapeutic treatment includes the inhibition of the progression of a condition, either physically by, e.g., stabilization of a discernible symptom, physiologically by, e.g., stabilization of a physical parameter, or both. In other embodiments the therapeutic treatment includes the reduction or stabilization of a disease, disorder or condition.

In one embodiment, the disease, or disorder is pain, inflammation, infection, cataracts, allergies, age-related macular degeneration (AMD), diabetic retinopathy (DR), macular edema, diabetic macular edema (DME), cytomegalovirus (CMV), retinitis, retinitis pigmentosa, uveitis, dry-eye syndrome, keratitis, glaucoma, blepharitis, blephariconjunctivtis, ocular hypertension, conjunctivitis, cystinosis, vitreomacular adhesion, corneal neovascularisation, corneal ulcers and post-surgical ocular inflammations/wound healing.

### EXAMPLES (formulations without monoacrylate are not according to the invention)

### Example 1: Ovalbumin (OVA, molecular weight 42.7 kDa)

### 1.1 Materials

Poly(ethylene glycol) diacrylate (Mn = 700 Da, PEGDA 700), 1-[4-(2-hydroxyethoxy)-phenyl]-2-hydroxy-2- methyl-1-propanone (Irgacure 2959), phosphate-buffered saline (PBS) solution tablets and poly(ethylene glycol) methacrylate (Mn = 360 Da, PEGMA) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Albumin from chicken egg white i.e. Ovalbumin (OVA) was purchased from Sigma Aldrich (Basingstoke, UK). Silicone tubing (peroxide-cured, 0.6 mm internal diameter, 0.27 mm wall thickness) was purchased from Polymer System Technology, UK.

### 1.2 Preparation of O1, 02, 03 and 04 implants

| **Formulation Code** | **OVA loading (% w/w)** | **PLGA 75/25 (% w/w)** | **PEGDA 700 (% w/w)** | **PEGMA (% w/w)** | **Irgacure (% w/w)** |
|---|---|---|---|---|---|
| O1 | 30 | 5 | 64.7 | 0 | 0.30 |
| O2 | 30 | 5 | 58.2 | 6.5 | 0.30 |
| O3 | 30 | 5 | 51.8 | 12.9 | 0.30 |
| O4 | 30 | 5 | 45.3 | 19.4 | 0.30 |

5 mg of PLGA 7502 i.e. 75/25 (Corbion Purac Biomaterials, Gorinchem, The Netherlands) was dissolved in the mixture of either **(i)** 64.7 mg PEGDA 700 Da (**O1**) or **(ii)** 58.2 mg PEGDA 700 Da and 6.5 mg PEGMA 360 Da (**O2**) or **(iii)** 51.8 mg PEGDA 700 Da and 12.9 mg PEGMA 360 Da (**O3**) or **(iv)** 45.3 mg PEGDA 700 Da and 19.4 mg PEGMA 360 Da (**O4**) to prepare Solution A. 5 mg Irgacure 2559 (Sigma Aldrich, Basingstoke, UK) was dissolved in 1 ml PBS to prepare Solution B. 30 mg of albumin from chicken egg white i.e. Ovalbumin (Sigma Aldrich, Basingstoke, UK) was put into a 60 µL of Solution B in Eppendorf tube (Sarstedt, Nümbrecht, Germany) to prepare Solution C. Solution A was added to the Eppendorf tube. Solution C was added to Solution A slowly through Eppendorf tube wall with continuous stirring at 900 rpm for 15 minutes. The mixtures finally obtained was withdrawn into silicon tubes with internal diameter (ID) of 0.635 mm (Polymer System Technology, UK) and photocrosslinked using a UV light at 365 nm (Light Hammer^{®} 6, Heraeus Noblelight Fusion UV Inc., Gaithersburg, MD, USA). The intensity of the UV lightwas set as 50% and the silicone tubes were exposed to the UV light for 15 seconds (i.e. a total of 5 runs). The implants were then removed from the silicon tubes and left to dry in vacuum at 25°C for 4 hours. The rod-shaped implants were cut at each 7.5 mm length.

### 1.3 In vitro drug release setup

*In vitro* release was conducted by placing two (2) implants of O1 in a glass vial containing 2 mL of PBS (Phosphate buffered saline) with 0.05% w/w NaN₃ (pH 7.4 ± 0.2) as release media. The similar *in vitro* release was conducted for implants O2, O3 and O4. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). Sampling followed by complete replacement of the PBS medium was performed on Day 1 and weekly thereafter, i.e. Day 7, Day 14, Day 21, Day 28 and so on. The concentration of released drug molecule in the PBS samples was analyzed as described in the following section.

### 1.4 In vitro degradation study set up

*In vitro* degradation study was conducted by placing two (2) implants of O1 in a glass vial containing either 4mL of 10 mM NaOH as accelerated degradation condition media or 4 mL of PBS (Phosphate buffered saline) with 0.05% w/w NaN₂ (pH 7.4 ± 0.2) as ambient condition degradation media. The similar *in vitro* degradation was conducted for implants 02, 03 and 04 implants. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). Sampling followed by complete replacement of the degradation medium was performed on each time point. The accelerated condition degradation was performed for 30 days while ambient condition degradation was performed in parallel to *in vitro* drug release until release completeness. The samples were analyzed for wet weight and dry weight of implants after each time points.

### 1.5 Sample analysis

Analysis of Ovalbumin (OVA) *in vitro* release samples were performed using SEC- HPLC with UV detection (Agilent 1260 Infinity Quaternary System, Agilent Ltd., UK) using a Phenomenex^{®} Biosep- SEC-S3000 column (300 mm length, 7.8 mm internal diameter and 5 µm particle size) and a Phenomenex^{®} SecurityGuard Cartridges GFC 3000 (4 × 3.0 mm ID) (Phenomenex, Torrance, USA). 20 µL of samples were eluted with 27 mM Phosphate buffer and 150 mM NaCl pH 6.35 with flowrate of 1.0 ml/min for 14 min. Detection was carried out by UV detector at 214 nm.

### 1.6 Results

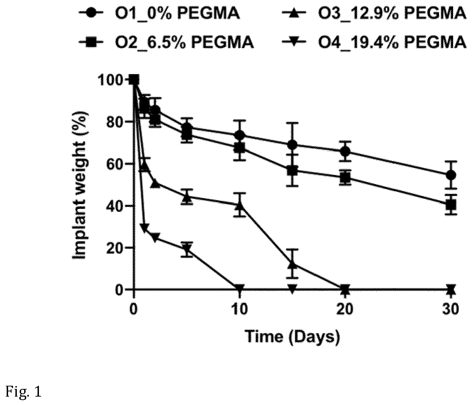

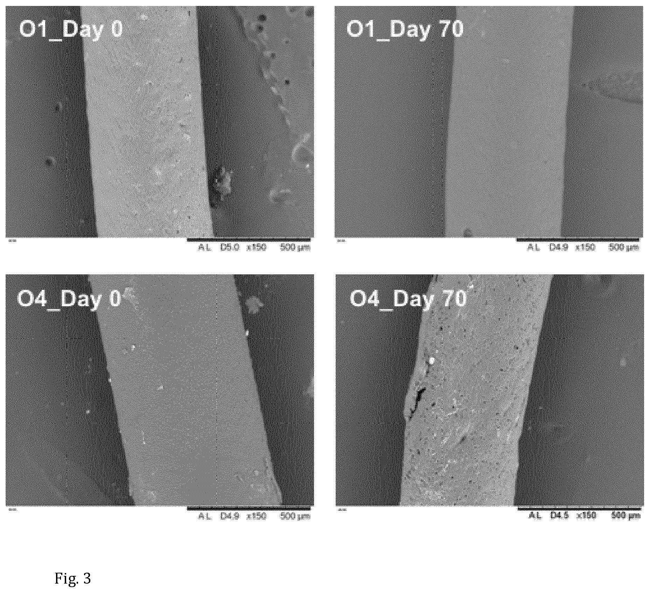

Figure 1 shows accelerated degradation profiles of O1, 02, 03 and 04 implants. The more the PEGMA polymer content in the implant, the faster the degradation of the implants. The SEM images in Figure 3 show that, while 04 degrades faster (decrease in diameter) than O1 and shows signs of surface erosion and pore formation, its physical structure remains intact during the degradation process. This enables to avoids the formation of debris in the front and/or the back of the eye, with the potential risk to clog the trabecular meshwork and to prevent proper drainage of the aqueous humors from the eye. Furthermore, the maintenance of the physical structure during degradation will prevent second and third burst effects from occurring, thus enabling a more sustained and predictable drug release over time, while preventing harmful overdoses, particularly in case of highly potent drugs.

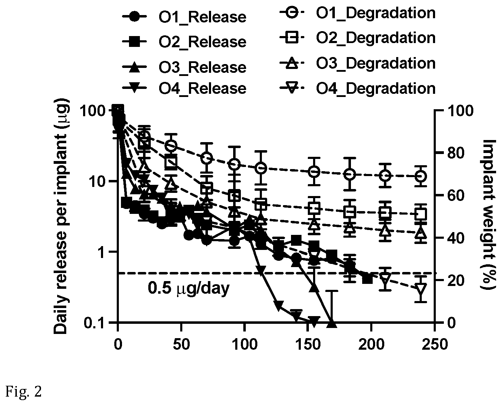

Figure 2 shows the daily OVA release rates and the degradation profile of implants O1, 02, 03 and 04. The minimum therapeutic level of 0.5 µg/day shown in Figure 2 is five times the IC₅₀ (minimum inhibitory concentration) value of Ranibizumab (LUCENTISO) (Genentech Inc. Prescribing Information for Lucentis^{®} (Ranibizumab), 2006; J. Gaudreault, D. Fei, J. Rusit, P. Suboc, V. Shiu, Preclinical pharmacokinetics of ranibizumab (rhuFabV2) after a single intravitreal administration, Investig. Ophthalmol. Vis. Sci. 46 (2005) 726-733). Ranibizumab has a molecular weight closed to Ovalbumin. Implant 04 degrades faster than implant O1. It nevertheless releases OVA in an acceptable therapeutic amount for more than 4 months.

### Example 2: Ovalbumin (OVA), molecular weight 42.7 kDa

### 2.1 Materials

Poly(ethylene glycol) diacrylate (Mn = 700 Da, PEGDA 700), 1-[4-(2-hydroxyethoxy)-phenyl]-2-hydroxy-2- methyl-1-propanone (Irgacure 2959), phosphate-buffered saline (PBS) solution tablets, poly(ethylene glycol) methacrylate (Mn = 360 Da, PEGMA), poly(ethylene glycol) methyl ether methacrylate (PEGMEMA, Mn 500 Da), poly(ethylene glycol) methyl ether acrylate (PEGMEA, Mn 480 Da), and poly(ethylene glycol) dimethacrylate (PEGDMA, Mn 750 Da) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Albumin from chicken egg white i.e. Ovalbumin (OVA) was purchased from Sigma Aldrich (Basingstoke, UK). Silicone tubing (peroxide-cured, 0.6 mm internal diameter, 0.27 mm wall thickness) was purchased from Polymer System Technology, UK.

### 2.2 Preparation of OV 1, OV 2, OV 3, OV 4 and OV 6 implants

| **Formulatio n Code** | **OVA loading (% w/w)** | **PLGA 7525 (% w/w)** | **Diacrylate (Name, % w/w)** | **Monoacrylate (Name, % w/w)** | **Irgacure (% w/w)** |
|---|---|---|---|---|---|
| OV 1 | 30 | 1 | PEGDA 700, 68.5 | 0 | 0.50 |
| OV 2 | 30 | 1 | PEGDA 700, 49.1 | PEGMA, 19.4 | 0.50 |
| OV 3 | 30 | 1 | PEGDA 700, 49.1 | PEGMEMA, 19.4 | 0.50 |
| OV 4 | 30 | 1 | PEGDA 700, 49.1 | PEGMEA, 19.4 | 0.50 |
| OV 6 | 30 | 1 | PEGDMA 750, 49.1 | PEGMA, 19.4 | 0.50 |

1 mg of PLGA 7502 i.e. 75/25 (Corbion Purac Biomaterials, Gorinchem, The Netherlands) and 0.5 mg of Irgacure 2959 (Sigma Aldrich, Basingstoke, UK) were dissolved in the mixture of either **(i)** 68.5 mg PEGDA 700 Da **(OV 1)** or **(ii)** 49.1 mg PEGDA 700 Da and 19.4 mg PEGMA 360 Da **(OV 2)** or **(iii)** 49.1 mg PEGDA 700 Da and 19.4 mg PEGMEMA 500 Da **(OV 3)** or **(iv)** 49.1 mg PEGDA 700 Da and 19.4 mg PEGMEA 480 Da **(OV 4)** or **(v)** 49.1 mg PEGDMA 750 Da and 19.4 mg PEGMA 360 Da **(OV 6)** to prepare Solution A. 30 mg of albumin from chicken egg white i.e. Ovalbumin (Sigma Aldrich, Basingstoke, UK) were put into a 45 µL of ultrapure water in Eppendorf tube (Sarstedt, Nümbrecht, Germany) to prepare Solution B. Solution A was added to the Eppendorf tube and Solution B was added to Solution A slowly through the Eppendorf tube wall with continuous stirring at 600 rpm for 10 minutes, followed by stirring at 300 rpm for another 30 minutes. The mixture so obtained was withdrawn into silicon tubes with internal diameter (ID) of 0.635 mm (Polymer System Technology, UK) and photocrosslinked using a UV light at 365 nm (Light Hammer^{®} 6, Heraeus Noblelight Fusion UV Inc., Gaithersburg, MD, USA). The intensity of the UV light was set as 50% and the silicone tubes were exposed to the UV light for 15 seconds (i.e. a total of 5 runs). The implants were then removed from the silicon tubes and left to dry in vacuum at 25°C for 4 hours. The rod-shaped implants were cut at each 5 mm length.

### 2.3 In vitro drug release setup

*In vitro* release was conducted by placing two (2) implants of OV 1 in a glass vial containing 2 mL of PBS (Phosphate buffered saline) with 0.05% w/w NaN₃ (pH 7.4 ± 0.2) as release media. The similar *in vitro* release was conducted for implants OV 2, OV 3, OV 4 and OV 6. All the experiments were carried out in triplicate. The glass vials containing implants were placed in an incubator and incubated at 37°C and 40 rpm (GFL Orbital Shaking Incubator; Gesellschaft für Labortechnik mbH, Germany). Sampling followed by complete replacement of the PBS medium was performed on Day 1 and weekly thereafter, i.e. Day 7, Day 14, Day 21, Day 28 and so on. The concentration of released drug molecule in the PBS samples was analyzed as described in the following section.

### 2.4 In vitro degradation study set up

*In vitro* degradation study was conducted by placing two (2) implants of OV 1 in a glass vial containing either 4 mL of 10 mM NaOH as accelerated degradation condition media or 4 mL of PBS (Phosphate buffered saline) with 0.05% w/w NaN₃ (pH 7.4 ± 0.2) as ambient condition degradation media. The similar *in vitro* degradation was conducted for OV 2, OV 3, OV 4 and OV 6 implants. All the experiments were carried out in triplicate. The glass vials containing implants were placed in an incubator and incubated at 37°C and 40 rpm (GFL Orbital Shaking Incubator; Gesellschaft für Labortechnik mbH, Germany). Sampling followed by complete replacement of the degradation medium was performed on each time point. The accelerated condition degradation was performed for 30 days while ambient condition degradation was performed in parallel to *in vitro* drug release until release completeness. The samples were analyzed for wet weight, dry weight, wet diameter and dry diameter of implants after each time points.

### 2.5 Sample analysis

Analysis of Ovalbumin (OVA) *in vitro* release samples were performed using SEC- HPLC with UV detection (Agilent 1260 Infinity Quaternary System, Agilent Ltd., UK) using a Phenomenex^{®} Biosep- SEC-S3000 column (300 mm length, 7.8 mm internal diameter and 5 µm particle size) and a Phenomenex^{®} SecurityGuard Cartridges GFC 3000 (4 × 3.0 mm ID) (Phenomenex, Torrance, USA). 20 µL of samples were eluted with 27 mM Phosphate buffer and 150 mM NaCl pH 6.35 with flowrate of 1.0 ml/min for 14 min. Detection was carried out by UV detector at 214 nm.

### 2.6 Results

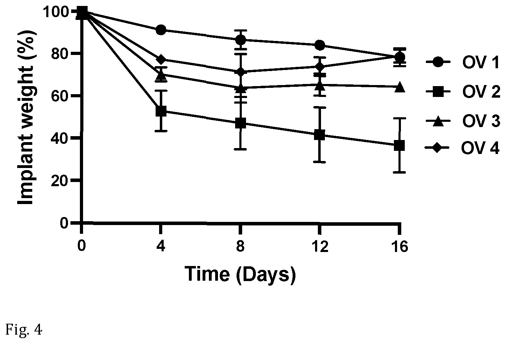

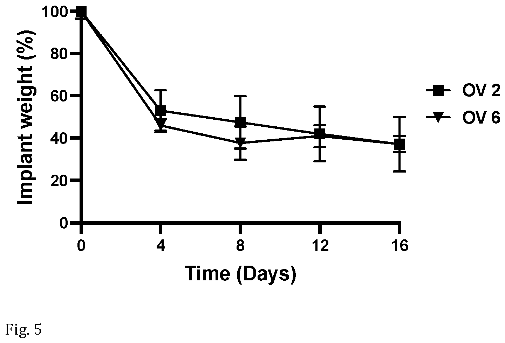

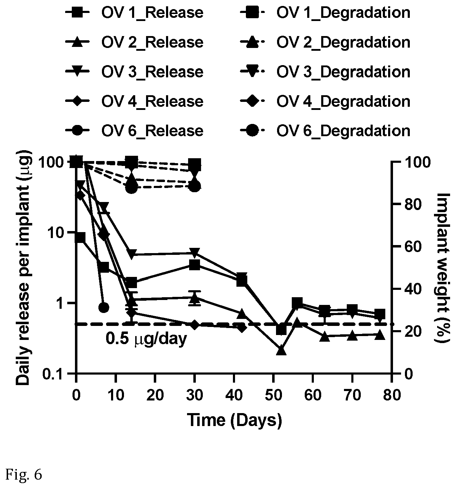

Figure 4 shows accelerated degradation profiles of OV 1 to OV 4 implants. The degradation profiles indicates that the use of various monoacrylate polymers in the implant matrix increases rate of degradation. Figure 5 showed similar degradation profiles of OV 2 and OV 6 implants, indicating no beneficial effect of use of another diacrylate i.e. polyethylene glycol) dimethacrylate in enhancing degradation of implants. Thus, the use of the monoacrylate polymers are responsible for enhancing degradation rate of implants. Figure 6 showed that the use of the monoacrylates like polyethylene glycol) methacrylate, PEGMA (OV 2) or poly(ethylene glycol) methyl ether methacrylate, PEGMEMA (OV 3) increases degradation rate of implants while maintaining drug release rate above minimum therapeutic level for 56 days or more.

### Example 3: Dexamethasone (DEX), molecular weight 392.46 Da

### 3.1 Materials

Poly(ethylene glycol) diacrylate (Mn = 250 Da, PEGDA 700), 1-[4-(2-hydroxyethoxy)-phenyl]-2-hydroxy-2- methyl-1-propanone (Irgacure 2959), phosphate-buffered saline (PBS) solution tablets, poly(ethylene glycol) methacrylate (Mn = 360 Da, PEGMA) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Dexamethasone (DEX) was purchased from Alfa Aesar (Heysham, UK). Silicone tubing (peroxide-cured, 0.3 mm internal diameter, 0.17 mm wall thickness) was purchased from Polymer System Technology, UK.

### 3.2 Preparation of DEX 1. DEX 2 and DEX 3 implants

| **Formulation Code** | **DEX loading (% w/w)** | **PLGA 7525 (% w/w)** | **PEGDA 250 (% w/w)** | **PEGMA 360 (% w/w)** | **Irgacure (% w/w)** |
|---|---|---|---|---|---|
| DEX 1 | 10 | 5 | 84.5 | 0 | 0.50 |
| DEX 2 | 10 | 5 | 74.5 | 10 | 0.50 |
| DEX 3 | 10 | 5 | 64.5 | 20 | 0.50 |

For a total mixture of 100 mg per implant, 10 mg of DEX (Alfa Aesar, Heysham, UK) was mixed with 5 mg of PLGA 75/25 (Corbion Purac Biomaterials, Gorinchem, The Netherlands) and added with **(i)** 84.5 mg of PEGDA 250 (**DEX 1**) or **(ii)** 74.5 mg PEGDA 250 with 10 mg PEGMA 360 (**DEX 2**) or **(iii)** 64.5 mg PEGDA 250 with 20 mg PEGMA 360 (**DEX 3**). The mixture was stirred at 200 rpm for 24 hr (Multistirrer, Velp ScientificaTM, Italy). Finally 0.5 mg of Irgacure 2959 was added to each mixture and stirred for another 10 minutes. The mixtures finally obtained was withdrawn into silicon tubes with internal diameter (ID) of 0.300 mm (Polymer System Technology, UK) and photocrosslinked using a UV light at 365 nm (Light Hammer^{®} 6, Heraeus Noblelight Fusion UV Inc., Gaithersburg, MD, USA). The intensity of the UV light was set as 100% and the silicone tubes were exposed to the UV light for 30 seconds (i.e. a total of 10 runs). The rod-shaped implants were cut at each 5 mm length.

### 3.3 In vitro drug release setup

*In vitro* release was conducted by placing four (4) implants of DEX 1 in a glass vial containing 4 mL of PBS (Phosphate buffered saline) with 0.05% w/w NaN₃ (pH 7.4 ± 0.2) as release media. The similar *in vitro* release was conducted for implants DEX 2 and DEX 3. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). On sampling timepoints of Day 1 and weekly thereafter, i.e., Day 7, Day 14, Day 21, Day 28 and so on, 1 mL of PBS was withdrawn and replaced with 1 mL of fresh PBS. The concentration of released drug molecule in the PBS samples was analyzed as described in the following section.

### 3.4 In vitro degradation study set up

*In vitro* degradation study was conducted by placing four (4) implants of DEX 1 in a glass vial containing either 4 mL of 50 mM NaOH as accelerated degradation condition media or 4 mL of PBS (Phosphate buffered saline) with 0.05% w/w NaN₃ (pH 7.4 ± 0.2) as ambient condition degradation media. The similar *in vitro* degradation was conducted for DEX 2 and DEX 3 implants. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). Sampling followed by complete replacement of the degradation medium was performed on each time point. The accelerated condition degradation was performed for 16 days while ambient condition degradation was performed in parallel to *in vitro* drug release until release completeness. The samples were analyzed for wet weight, dry weight, wet diameter and dry diameter of implants after each time points.

### 3.5 Sample analysis

Analysis of Dexamethasone (DEX) *in vitro* release samples were performed using VWD- HPLC with UV detection (Agilent 1260 Infinity Quaternary System, Agilent Ltd., UK) using a Poroshell 120 EC-C18, 4 µm (250 × 4.60 mm) analytical column fitted with a refillable guard column (Agilent, UK). The mobile phase consisted of acetonitrile and water in the ratio 40:60. The mobile phase was filtered by passing through 0.45 µm membrane filter (Whatman International, UK) under vacuum and degassed before use. The flow rate of mobile phase was 0.8 mL/min and the eluted drug was detected at 245 nm wavelength. Chromatographic separation of the DEX was achieved at ambient room temperature (24±2°C).

### 3.6 Results

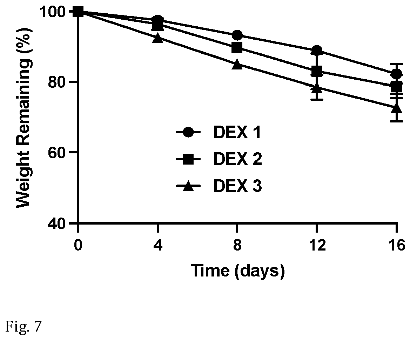

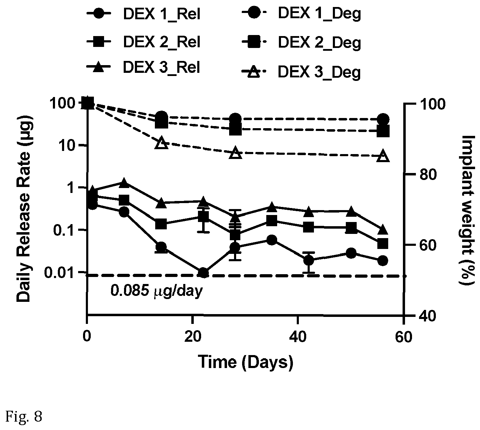

Figure 7 shows accelerated degradation profiles of DEX 1, DEX 2 and DEX 3 implants in 50 mM NaOH at 37°C and shaking speed of 40 rpm. The degradation profiles indicate that the use of the monoacrylate polymers in the implant matrix increases the rate of degradation. Figure 8 shows daily DEX release rate (in µg) per implant vs. implant weight loss from DEX 1, DEX 2 and DEX 3 implants. It indicates that the use of the monoacrylate polymer is responsible for enhancing degradation rate of implants while maintaining drug release rate above minimum therapeutic level for 56 days and beyond. (Nehmé A, Lobenhofer EK, Stamer WD, Edelman JL. Glucocorticoids with different chemical structures but similar glucocorticoid receptor potency regulate subsets of common and unique genes in human trabecular meshwork cells. BMC Med Genomics. 2009 Sep 10;2:58).

### Example 4: Latanoprost (LP), molecular weight 432 Da

### 4.1 Materials

Poly(ethylene glycol) diacrylate (Mn = 250 Da, PEGDA 700), 1-[4-(2-hydroxyethoxy)-phenyl]-2-hydroxy-2- methyl-1-propanone (Irgacure 2959), phosphate-buffered saline (PBS) solution tablets, poly(ethylene glycol) methacrylate (Mn = 360 Da, PEGMA) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Latanoprost (LP) was purchased from Alfa Chemistry, New York, US. Silicone tubing (peroxide-cured, 0.3 mm internal diameter, 0.17 mm wall thickness) was purchased from Polymer System Technology, UK.

### 4.2 Preparation of LP 1, LP 2 and LP 3 implants

| **Formulation Code** | **LP loading (% w/w)** | **PLGA 7525 (% w/w)** | **PEGDA 250 (% w/w)** | **PEGMA 360 (% w/w)** | **Irgacure (% w/w)** |
|---|---|---|---|---|---|
| LP 1 | 20 | 5 | 74.5 | 0 | 0.50 |
| LP 2 | 20 | 5 | 64.5 | 10 | 0.50 |
| LP 3 | 20 | 5 | 54.5 | 20 | 0.50 |

20 mg Irgacure 2959 (Sigma Aldrich, Basingstoke, England) was dissolved in acetonitrile to prepare Solution A. 50 mg Latanoprost (LP) (Alfa Chemistry, New York, USA) was dissolved in 0.5 mL acetonitrile to prepare Solution B. For the preparation for 250 mg LP-polymer mixture, **(i)** 186.25 mg PEGDA 250 and 12.5 mg of PLGA 75/25 (Purac Biochem, Gorinchem, The Netherlands) (**LP 1**) or **(ii)** 161.25 mg PEGDA 250, 25 mg PEGMA 360 and 12.5 mg of PLGA 75/25 (Purac Biochem, Gorinchem, The Netherlands) (**LP 2**) or **(iii)** 136.25 mg PEGDA 250, 50 mg PEGMA 360 and 12.5 mg of PLGA 75/25 (Purac Biochem, Gorinchem, The Netherlands) (**LP 3**) were put into a 2 mL Eppendorf tube, and dissolved in 250 µL acetonitrile to prepare Solution C. 62.5 µL of Solution A and 0.5 mL of Solution B were then added to Solution C, and subsequently stirred at 250 rpm for 30 minutes (Multistirrer, Velp ScientificaTM, Italy). Acetonitrile was then evaporated under gauge pressure of-0.1 MPa at room temperature for 6 h (OV-12 vacuum oven; JeioTech, Korea). The mixture finally obtained was withdrawn into a silicon tube of internal diameter (ID) of 0.32 mm (Polymer System Technology, UK) by using a 25G needle attached to 1 mL syringe and photocrosslinked using a UV light at 365 nm (Light Hammer^{®} 6, Heraeus Noblelight Fusion UV Inc., Gaithersburg, MD, USA). The intensity of the UV light was set as 100% and the silicone tubes were exposed to the UV light for 30 seconds (i.e. a total of 10 runs). The rod-shaped implants were cut at each 5 mm length.

### 4.3 In vitro drug release setup

*In vitro* release was conducted by placing four (4) implants of LP 1 in a glass vial containing 4 mL of PBS (Phosphate buffered saline) with 0.05% w/w NaN₃ (pH 7.4 ± 0.2) as release media. The similar *in vitro* release was conducted for implants LP 2 and LP 3. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). On sampling timepoints of Day 1 and weekly thereafter, i.e. Day 7, Day 14, Day 21, Day 28 and so on, 1 mL of PBS was withdrawn and replaced with 1 mL of fresh PBS. The concentration of released drug molecule in the PBS samples was analyzed as described in the following section.

### 4.4 In vitro degradation study set up

*In vitro* degradation study was conducted by placing four (4) implants of LP 1 in a glass vial containing either 4 mL of 50 mM NaOH as accelerated degradation condition media or 4 mL of PBS (Phosphate buffered saline) with 0.05% w/w NaN₃ (pH 7.4 ± 0.2) as ambient condition degradation media. The similar *in vitro* degradation was conducted for LP 2 and LP 3 implants. All the experiments were carried out in triplicate. The glass vials containing implants were placed in an incubator and incubated at 37°C and 40 rpm (SciQuip FL16-2 Refrigerated Incubating shaker, SciQuip Ltd. Shropshire, UK). Sampling followed by complete replacement of the degradation medium was performed on each time point. The accelerated condition degradation was performed for 16 days while ambient condition degradation was performed in parallel to *in vitro* drug release until release completeness. The samples were analyzed for wet weight, dry weight, wet diameter and dry diameter of implants after each time points.

### 4.5 Sample analysis

Analysis of LP1, LP2 and LP3 samples was performed using HPLC system with fluorescence detection (Agilent 1260 Infinity II Quaternary System) using a Poroshell 120 EC-C18 column (250 mm length, 4.6 mm internal diameter and 4 µm particle size). The samples were analyzed in an isocratic mode using a mobile phase of acetonitrile: 0.1% v/v formic acid (60:40), with an injection volume of 50 µL and a flow rate of 1 mL/min. The column temperature was maintained at 40°C. The fluorescence detector was set at an excitation wavelength of 265 nm and an emission wavelength of 285 nm.

### 4.6 Results

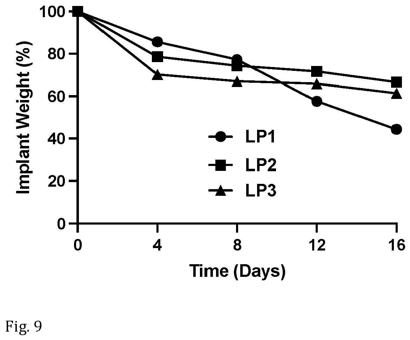

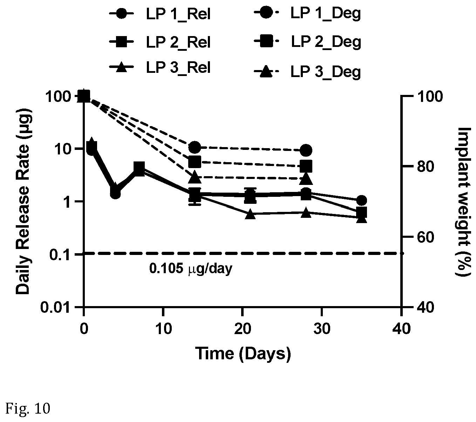

Figure 9 shows accelerated degradation profiles of LP 1, LP 2 and LP 3 implants in 50 mM NaOH at 37°C and shaking speed of 40 rpm. The degradation profiles indicate that the use of a monoacrylate polymer in the implant matrix increases the rate of degradation. Figure 10 shows daily LP release rate (in µg) per implant vs. implant weight loss from LP 1, LP 2 and LP 3 implants. It indicates that the use of the monoacrylate polymer is responsible for enhancing the degradation rate of implants while maintaining drug release rate above the minimum therapeutic level of 0.105 µg/day (Sharif, N.A., Kelly, C.R., Crider, J.Y.; Agonist activity of bimatoprost, travoprost, latanoprost, unoprostone isopropyl ester and other prostaglandin analogs at the cloned human ciliary body FP prostaglandin receptor; Journal of Ocular Pharmacology and Therapeutics, (2002); 18: 313-324) for at least 35 days.

### Example 5: Bevacizumab (BEZ), molecular weight 150 kDa

### 5.1 Materials

Poly(ethylene glycol) diacrylate (Mn = 700 Da, PEGDA 700), 1-[4-(2-hydroxyethoxy)-phenyl]-2-hydroxy-2- methyl-1-propanone (Irgacure 2959), phosphate-buffered saline (PBS) solution tablets, poly(ethylene glycol) methacrylate (Mn = 360 Da, PEGMA) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Bevacizumab (BEZ) (Avastin^{®}) was purchased from local pharmacy (manufactured by Roche, Switzerland; each vial consists of 100 mg BEZ in 4 mL i.e., 25 mg/ml) Silicone tubing (peroxide-cured, 0.635 mm internal diameter, 0.27 mm wall thickness) was purchased from Polymer System Technology, UK.

### 5.2 Preparation of F19, B6 and B7 implants

| **Formulation Code** | **BEZ loading (% w/w)** | **PLGA 7525 (% w/w)** | **PEGDA 700 (% w/w)** | **PEGMA 360 (% w/w)** | **Irgacure (% w/w)** |
|---|---|---|---|---|---|
| F19 | 50 | 5 | 44.25 | 0 | 0.75 |
| B6 | 50 | 5 | 39.25 | 5 | 0.75 |
| B7 | 50 | 5 | 42.05 | 2.21 | 0.75 |

For preparation of the polymer mixture, 50 mg PLGA 7502 (75/25) were dissolved in **(i)** 442.5 mg PEGDA 700 (**F19**) or **(ii)** 392.5 mg PEGDA 700 and 50 mg PEGMA 360 (**B6**) or **(iii)** 420.5 mg PEGDA 700 and 22.1 mg PEGMA 360 (**B7**) to prepare Solution A. 6 mg Irgacure 2559 were dissolved in 1 mL PBS (0.01 M, pH 7.4) to prepare Solution B. 62.5 µL of Solution B was put into a 2 mL Eppendorf tube. 25 mg BEZ were subsequently added to the Eppendorf tube and stirred with a stirrer bar at 250 rpm for 30 seconds. The mixture so obtained was left at 4-7°C for 30 minutes to dissolve and it was subsequently centrifuged for 1 minute at 2000 rpm to remove air bubbles. The so obtained mixture was added with 25 mg of Solution A and subsequently stirred at 250 rpm for 30 minutes. It was then centrifuged for 1 minute at 2000 rpm to remove air bubbles and subsequently stirred at 250 rpm for another 10 minutes. The mixture finally obtained was withdrawn into a silicon tube (ID: 0.635 mm) and crosslinked using a light hammer (Light Hammer^{®} 6, Heraeus Noblelight Fusion UV Inc., Gaithersburg, MD, USA). The intensity of the UV light (365 nm) was set as 50% and the silicone tubes were exposed to the UV light for 15 sec (5 runs). The rod shape implants were removed from the silicon tube and left to dry in vacuum at 25°C for 4 hours. The dried implants were cut into a 5 mm length.

### 5.3 In vitro drug release study

For drug release studies, one implant of each F19, B6 and B7 was placed into a glass vial containing 1 ml PBS (pH 7.4, 0.05% NaN₃). The glass vials were placed in a static incubator at 37°C. Sampling followed by complete replacement of the PBS release medium was performed on Day 1 and weekly thereafter, i.e. Day 7, Day 14, Day 21, Day 28 and so on. All the experiments were carried out in triplicates. The concentration of released drug molecule in the PBS samples were analyzed as described below.

### 5.4 SEC-HPLC analysis method for BEZ

Analysis of released bevacizumab (BEZ) from B6 and F19 implants were performed using SEC-HPLC with fluorescence detection (Agilent 1260 Infinity Quaternary System) using a Phenomenex^{®} BioZen^{™} SEC-2 column (150 mm length, 4.6 mm internal diameter and 1.8 µm particle size) and a Phenomenex^{®} BioZen^{™} SEC-2 0.46 mm SecurityGuard Ultra Cartridge (Phenomenex, Torrance, USA). BEZ samples were analyzed in an isocratic mode using a mobile phase of 35 mM sodium phosphate buffer (pH 6.8, 300 mM NaCl), with an injection volume of 10 µL and a flow rate of 0.5 mL/min. The column temperature was maintained at 25°C. The fluorescence detector was set at an excitation wavelength of 280 nm and an emission wavelength of 340 nm.

### 5.5 Micro BCA analysis method for BEZ

Analysis of released bevacizumab (BEZ) from B7 implants using Micro BCA protein assay kit (Pierce Biotechnology, Thermofisher Scientific, USA). Briefly 150 µL of sample was mixed with 150 µL of Micro BCA reagent mixture and incubated at 37°C for 2 hr. The intensity of colour developed due to the interaction between protein and Micro BCA reagent was measured at wavelength of 562 nm.

### 5.6 In vitro degradation study set up

*In vitro* degradation study was conducted by placing one implant of F19 in a glass vial containing either 4 mL of 10 mM NaOH as accelerated degradation condition media or 4 mL of PBS (phosphate buffered saline) with 0.05% w/w NaN₃ (pH 7.4 ± 0.2) as ambient condition degradation media. The similar *in vitro* degradation was conducted for B6 and B7 implants. All the experiments were carried out in triplicate. The glass vials containing implants were placed in a static incubator and incubated at 37°C (MINI/100/F, Genlab Limited, Cheshire, UK). Sampling followed by complete replacement of the degradation medium was performed on each time point The accelerated condition degradation was performed for 28 days while ambient condition degradation was performed in parallel to *in vitro* drug release until release completeness. The samples were analyzed for wet weight, dry weight, wet diameter and dry diameter of implants after each time points.

### 5.7 Results

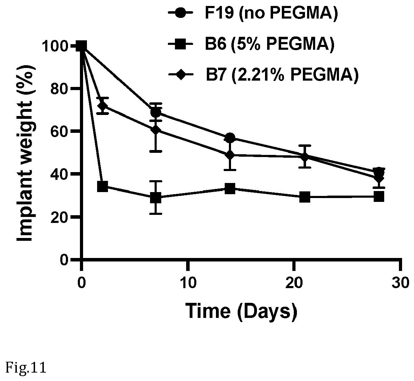

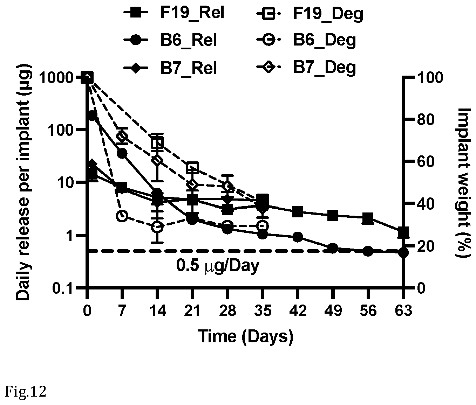

Figure 11 shows accelerated degradation profiles of F19, B6 and B7 implants in 10 mM NaOH at 37°C. The degradation profiles indicates that the use of the monoacrylate polymer in the implant matrix increases rate of degradation. Figure 12 shows daily BEZ release rate (in µg) per implant vs. implant weight loss from F19, B6 and B7 implants. It indicates that the use of the monoacrylate polymer is responsible for enhancing the degradation rate of implants while maintaining the drug release rate above the minimum target therapeutic level of 0.5 µg/day (about 5 times the IC50 value, WangY, Fei D, Vanderlaan M, Song A. Angiogenesis. 2004;7:335) for at least 56 days (for B6 implants).

### Example 6: Ovalbumin (OVA, molecular weight 42.7 kPa)

### 6.1 Materials

Poly(ethylene glycol) diacrylate (Mn = 700 Da, PEGDA 700), 1-[4-(2-hydroxyethoxy)-phenyl]-2-hydroxy-2- methyl-1-propanone (Irgacure 2959), phosphate-buffered saline (PBS) solution tablets and poly(ethylene glycol) methacrylate (Mn = 360 Da, PEGMA) were purchased from Sigma (Dorset, UK). Poly(lactide-co-glycolide) (PURASORB^{®} PDLG 7502, 75:25, PLGA 75/25) was obtained from Corbion Purac Biomaterials (Gorinchem, The Netherlands). Albumin from chicken egg white i.e. Ovalbumin (OVA) was purchased from Sigma Aldrich (Basingstoke, UK). Silicone tubing (peroxide-cured, 0.6 mm internal diameter, 0.27 mm wall thickness) was purchased from Polymer System Technology, UK.

### 6.2 Preparation of B1, B2 and B3 implants

| **Formulation Code** | **OVA loading (% w/w)** | **PLGA 75/25 (% w/w)** | **PEGDA 700 (% w/w)** | **PEGMA (% w/w)** | **Irgacure (% w/w)** |
|---|---|---|---|---|---|
| B1 | 40 | 5 | 35.1 | 19.4 | 0.5 |
| B2 | 40 | 5 | 30.5 | 24 | 0.5 |
| B3 | 40 | 5 | 26.5 | 28 | 0.5 |

5 mg of PLGA 7502 i.e. 75/25 (Corbion Purac Biomaterials, Gorinchem, The Netherlands) was dissolved in the mixture of either **(i)** 35.1 mg PEGDA 700 Da and 19.4 mg PEGMA 360 Da (**B1**) or **(ii)** 30.5 mg PEGDA 700 Da and 24 mg PEGMA 360 Da (**B2**) or **(iii)** 26.5 mg PEGDA 700 Da and 28 mg PEGMA 360 Da (**B3**) to prepare Solution A. 5 mg Irgacure 2559 (Sigma Aldrich, Basingstoke, UK) was dissolved in 1 ml PBS to prepare Solution B. 40 mg of albumin from chicken egg white i.e. Ovalbumin (Sigma Aldrich, Basingstoke, UK) was put into a 60 µL of Solution B in Eppendorf tube (Sarstedt, Nümbrecht, Germany) to prepare Solution C. Solution A was added to the Eppendorf tube. Solution C was added to Solution A slowly through Eppendorf tube wall with continuous stirring at 600 rpm for 15 minutes. The mixtures finally obtained was withdrawn into silicon tubes with internal diameter (ID) of 0.635 mm (Polymer System Technology, UK) and photocrosslinked using a UV light at 365 nm (Light Hammer^{®} 6, Heraeus Noblelight Fusion UV Inc., Gaithersburg, MD, USA). The intensity of the UV light was set as 50% and the silicone tubes were exposed to the UV light for 15 seconds (i.e. a total of 5 runs). The implants were then removed from the silicon tubes and left to dry in vacuum at 25°C for 4 hours. The rod-shaped implants were cut at each 5 mm length.

### 6.3 In vitro drug release setup

*In vitro* release was conducted by placing two (2) implants of B1 in a glass vial containing 2 mL of PBS (Phosphate buffered saline) with 0.05% w/w NaN₃ (pH 7.4 ± 0.2) as release media. The similar *in vitro* release was conducted for implants B2 and B3. All the experiments were carried out in triplicate. The glass vials containing implants were placed in an incubator and incubated at 37°C and 40 rpm (GFL Orbital Shaking Incubator; Gesellschaft für Labortechnik mbH, Germany). Sampling followed by complete replacement of the PBS medium was performed on Day 1 and weekly thereafter, i.e. Day 7, Day 14, Day 21, Day 28 and so on. The concentration of released drug molecule in the PBS samples was analyzed as described in the following section.

### 6.4 In vitro degradation study set up

*In vitro* degradation study was conducted by placing two (2) implants of B1 in a glass vial containing either 4 mL of 10 mM NaOH as accelerated degradation condition media or 4 mL of PBS (Phosphate buffered saline) with 0.05% w/w NaN₃ (pH 7.4 ± 0.2) as ambient condition degradation media. The similar *in vitro* degradation was conducted for implants B2 and B3 implants. All the experiments were carried out in triplicate. The glass vials containing implants were placed in an incubator and incubated at 37°C and 40 rpm (GFL Orbital Shaking Incubator; Gesellschaft für Labortechnik mbH, Germany). Sampling followed by complete replacement of the degradation medium was performed on each time point The accelerated condition degradation was performed for 30 days while ambient condition degradation was performed in parallel to *in vitro* drug release until release completeness. The samples were analyzed for wet weight and dry weight of implants after each time points.

### 6.5 Sample analysis

Analysis of Ovalbumin (OVA) *in vitro* release samples were performed using SEC- HPLC with UV detection (Agilent 1260 Infinity Quaternary System, Agilent Ltd., UK) using a Phenomenex^{®} Biosep- SEC-S3000 column (300 mm length, 7.8 mm internal diameter and 5 µm particle size) and a Phenomenex^{®} SecurityGuard Cartridges GFC 3000 (4 × 3.0 mm ID) (Phenomenex, Torrance, USA). 20 µL of samples were eluted with 27 mM Phosphate buffer and 150 mM NaCl pH 6.35 with flowrate of 1.0 ml/min for 14 min. Detection was carried out by UV detector at 214 nm.

### 6.6 Results

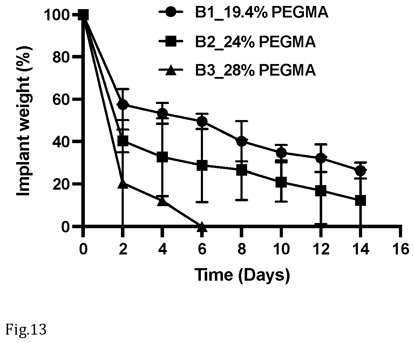

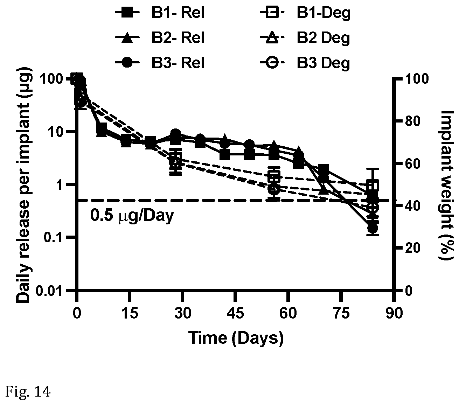

Figure 13 shows accelerated degradation profiles of B1, B2 and B3 implants. The more the PEGMA polymer content in the implant, the faster the degradation of the implants. Figure 14 shows the daily OVA release rates and the degradation profile of implants B1, B2 and B3. Higher PEGMA concentration in B3 implant lead to faster degradation of implant measured as % implant weight loss while maintaining release rate above minimum therapeutic level for at least 75 days.

## Claims

1. An ocular composition comprising:
a) at least 0.1% w/w of a therapeutic agent;
b) 5 to 95% w/w of a photopolymerizable composition comprising 3 to 70% w/w of one or more compounds of formula I: 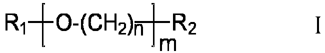
wherein R₁ is hydrogen or a linear or branched C₁-C₃ alkyl; R₂ is an acrylate or methacrylate group; n is 2 or 3 and m is equal or greater than 1, the weight percentage of the one or more compounds of formula I being based on the total weight of the photopolymerizable composition,
wherein the photopolymerizable composition further comprises one or more disubstituted acrylate or methacrylate compounds selected from the group consisting of ethylene glycol diacrylate, di (ethylene glycol) diacrylate, poly (ethylene glycol) diacrylate, ethylene glycol dimethacrylate, di (ethylene glycol) dimethacrylate, poly (ethylene glycol) dimethacrylate, propylene glycol diacrylate, di (propylene glycol) diacrylate, poly (propylene glycol) diacrylate, propylene glycol dimethacrylate, di (propylene glycol) dimethacrylate, poly (propylene glycol) dimethacrylate, 1,6-hexanediol dimethacrylate;
c) 0.1 to 40% w/w of a biodegradable polymer selected from the group consisting of lactide/glycolide copolymer (including poly(lactide-co-glycolide) (PLGA)), poly (L-lactide) (PLA), polyhydroxyalkanoates, including polyhydroxybutyrate, polyglycolic acid (PGA), polycaprolactone (PCL), poly (DL-lactide) (PDL), poly (D-lactide), lactide/caprolactone copolymer, poly-L-lactide-co-caprolactone (PLC) and mixtures, copolymers, and block copolymers thereof, and
d) a photoinitiator.

2. The ocular composition according to claim 1, wherein the compound of formula I is selected from the group consisting of poly(ethylene glycol) acrylate, polyethylene glycol) methacrylate, polyethylene glycol) methyl ether acrylate, poly(ethylene glycol) methyl ether methacrylate, ethylene glycol methyl ether acrylate, di(ethylene glycol) ethyl ether acrylate, ethylene glycol methyl ether methacrylate, di(ethylene glycol) ethyl ether methacrylate and mixtures thereof.

3. The ocular composition according to claim 2, wherein the compound of formula I is poly (ethylene glycol) methacrylate (PEGMA).

4. The ocular composition according to any preceding claim, wherein the photopolymerizable composition comprises 5 to 60% w/w of one or more compounds of formula I.

5. The ocular composition according to any preceding claim, wherein the one or more disubstituted acrylate or methacrylate compounds are selected from the group consisting of poly (ethylene glycol) diacrylate and poly (ethylene glycol) dimethacrylate.

6. The ocular composition according to claim 5, wherein the one or more disubstituted acrylate or methacrylate compounds is poly (ethylene glycol) diacrylate (PEGDA).

7. The ocular composition according to any preceding claim, comprising 40 to 75% w/w of the photopolymerizable composition.

8. The ocular composition according to any preceding claim, wherein the biodegradable polymer is lactide/glycolide copolymer (including poly(L-lactide-co-glycolide) (PLGA)), poly (L-lactide) (PLA), poly(DL-lactide) (PDL), and lactide/caprolactone copolymer (PLC).

9. The ocular composition according to claim 8, wherein the biodegradable polymer is poly(lactide-co-glycolide) (PLGA).

10. The ocular composition according to any preceding claim, wherein the therapeutic agent is present in an amount between 20 and 70% w/w.

11. The ocular composition according to any preceding claim, comprising
a) 10 to 50% w/w of the therapeutic agent
b) 1 to 20% w/w of poly(lactide-co-glycolide) (PLGA)
c) 30 to 88.99% w/w of the photopolymerizable composition consisting of poly (ethylene glycol) methacrylate (PEGMA) and poly (ethylene glycol) diacrylate (PEGDA).
d) 0.01 to 5% w/w of a photoinitiator selected from the group consisting of 1-[4-(2-hydroxyethoxy)-phenyl]-2-hydroxy-2- methyl-1-propanone, phenyl-bis(2,4,6-trimethylbenzoyl)- phosphineoxide and mixtures thereof.
wherein the weight ratio PEGDA:PEGMA is between 0.5 and 20, 0.5 and 15, 1 and 15, 1 and 10, or 2 and 5.

12. The ocular composition according to any preceding claim, wherein the therapeutic agent is ranibizumab, bevacizumab, latanoprost, dexamethasone or timolol maleate.

13. A method of making an ocular implant, comprising the steps of:
a) Providing an ocular composition according to any claim 1 to 12; and
b) Irradiating the ocular composition with light at a wavelength between 200 and 550 nm for a period of time between 1 second and 60 minutes to form the ocular implant.

14. An ocular implant comprising at least 0.1% w/w of a therapeutic agent, 5 to 95% w/w of a crosslinked polymer matrix and 0.1 to 40% w/w of a biodegradable polymer selected from the group consisting of lactide/glycolide copolymer (including poly(lactide-co-glycolide) (PLGA)), poly (L-lactide) (PLA), polyhydroxyalkanoates, including polyhydroxybutyrate, polyglycolic acid (PGA), polycaprolactone (PCL), poly (DL-lactide) (PDL), poly (D-lactide), lactide/caprolactone copolymer, poly-L-lactide-co-caprolactone (PLC) and mixtures, copolymers, and block copolymers thereof, **characterized in that**:
a) the crosslinked polymer matrix is obtained by crosslinking a photopolymerizable composition comprising 3 to 70% w/w of one or more compounds of formula I: 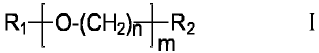
wherein R₁ is hydrogen or a linear or branched C₁-C₃ alkyl; R₂ is an acrylate or methacrylate group; n is 2 or 3 and m is equal or greater than 1, the weight percentage of the one or more compounds of formula I being based on the total weight of the photopolymerizable composition,
wherein the photopolymerizable composition further comprises one or more disubstituted acrylate or methacrylate compounds selected from the group consisting of ethylene glycol diacrylate, di (ethylene glycol) diacrylate, poly (ethylene glycol) diacrylate, ethylene glycol dimethacrylate, di (ethylene glycol) dimethacrylate, poly (ethylene glycol) dimethacrylate, propylene glycol diacrylate, di (propylene glycol) diacrylate, poly (propylene glycol) diacrylate, propylene glycol dimethacrylate, di (propylene glycol) dimethacrylate, poly (propylene glycol) dimethacrylate, 1,6-hexanediol dimethacrylate;
b) the therapeutic agent and the biodegradable polymer are embedded in the polymer matrix.

15. The ocular implant according to claim 14, comprising or consisting of:
a) 10 to 50% w/w of the therapeutic agent;
b) 1 to 20% w/w of poly(lactide-co-glycolide) (PLGA);
c) 30 to 89 % w/w of the crosslinked polymer matrix obtained by crosslinking the photopolymerizable composition consisting of poly (ethylene glycol) methacrylate (PEGMA) and poly (ethylene glycol) diacrylate (PEGDA), wherein the weight ratio PEGDA:PEGMA is between 0.5 and 20, 0.5 and 15, 1 and 15, 1 and 10, or 2 and 5.

## Patentansprüche

1. Okuläre Zusammensetzung, umfassend:
a) mindestens 0,1 Gew.-% eines therapeutischen Wirkstoffs;
b) 5 bis 95 Gew.-% einer photopolymerisierbaren Zusammensetzung, umfassend 3 bis 70 Gew.-% einer oder mehrerer Verbindungen der Formel I: 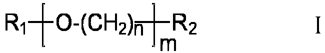
wobei R₁ Wasserstoff oder ein lineares oder verzweigtes C₁-C₃-Alkyl ist; R₂ eine Acrylat- oder Methacrylatgruppe ist; n 2 oder 3 ist und m gleich oder größer als 1 ist, wobei der Gewichtsprozentanteil der einen oder mehreren Verbindungen der Formel I auf das Gesamtgewicht der photopolymerisierbaren Zusammensetzung bezogen ist,
wobei die photopolymerisierbare Zusammensetzung weiter eine oder mehrere disubstituierte Acrylat- oder Methacrylatverbindungen umfasst, die aus der Gruppe ausgewählt sind, bestehend aus Ethylenglykoldiacrylat, Di(ethylenglykol)diacrylat, Poly(ethylenglykol)diacrylat, Ethylenglykoldimethacrylat, Di(ethylenglykol)dimethacrylat, Poly(ethylenglykol)dimethacrylat, Propylenglykoldiacrylat, Di(propylenglykol)diacrylat, Poly(propylenglykol)diacrylat, Propylenglykoldimethacrylat, Di(propylenglykol)dimethacrylat, Poly(propylenglykol)dimethacrylat, 1,6-Hexandioldimethacrylat;
c) 0,1 bis 40 Gew.-% eines biologisch abbaubaren Polymers, ausgewählt aus der Gruppe bestehend aus Lactid/Glycolid-Copolymer (einschließlich Poly(lactid-co-glycolid) (PLGA)), Poly(L-lactid) (PLA), Polyhydroxyalkanoaten, einschließlich Polyhydroxybutyrat, Polyglykolsäure (PGA), Polycaprolacton (PCL), Poly(DL-lactid) (PDL), Poly(D-lactid), Lactid/Caprolacton-Copolymer, Poly-L-lactid-co-Caprolacton (PLC) und Mischungen, Copolymeren und Blockcopolymeren davon, und
d) einen Photoinitiator.

2. Okuläre Zusammensetzung nach Anspruch 1, wobei die Verbindung der Formel I ausgewählt ist aus der Gruppe bestehend aus Poly(ethylenglykol)acrylat, Poly(ethylenglykol)methacrylat, Poly(ethylenglykol)methyletheracrylat, Poly(ethylenglykol)methylethermethacrylat, Ethylenglykolmethyletheracrylat, Di(ethylenglykol)ethyletheracrylat, Ethylenglykolmethylethermethacrylat, Di(ethylenglykol)ethylethermethacrylat und deren Mischungen.

3. Okuläre Zusammensetzung nach Anspruch 2, wobei die Verbindung der Formel I Poly(ethylenglykol)methacrylat (PEGMA) ist.

4. Okuläre Zusammensetzung nach einem vorstehenden Anspruch, wobei die photopolymerisierbare Zusammensetzung 5 bis 60 Gew.-% einer oder mehrerer Verbindungen der Formel I umfasst.

5. Okuläre Zusammensetzung nach einem vorstehenden Anspruch, wobei die eine oder mehreren disubstituierten Acrylat- oder Methacrylatverbindung(en) aus der Gruppe ausgewählt ist/sind, die aus Poly(ethylenglykol)diacrylat und Poly(ethylenglykol)dimethacrylat besteht.

6. Okuläre Zusammensetzung nach Anspruch 5, wobei es sich bei der einen oder den mehreren disubstituierten Acrylat- oder Methacrylatverbindung(en) um Poly(ethylenglykol)diacrylat (PEGDA) handelt.

7. Okuläre Zusammensetzung nach einem vorstehenden Anspruch, umfassend 40 bis 75 Gew.-% der photopolymerisierbaren Zusammensetzung.

8. Okuläre Zusammensetzung nach einem vorstehenden Anspruch, wobei das biologisch abbaubare Polymer ein Lactid/Glycolid-Copolymer ist (einschließlich Poly(L-lactid-co-glycolid) (PLGA)), Poly(L-lactid) (PLA), Poly(OL-lactid) (PDL) und Lactid/Caprolacton-Copolymer (PLC).

9. Okuläre Zusammensetzung nach Anspruch 8, wobei das biologisch abbaubare Polymer Poly(lactid-co-glycolid) (PLGA) ist.

10. Okuläre Zusammensetzung nach einem vorstehenden Anspruch, wobei der therapeutische Wirkstoff in einer Menge zwischen 20 und 70 Gew.-% vorhanden ist.

11. Okuläre Zusammensetzung nach einem vorstehenden Anspruch, umfassend
a) 10 bis 50 Gew.-% des therapeutischen Wirkstoffs
b) 1 bis 20 Gew.-% Poly(lactid-co-glycolid) (PLGA)
c) 30 bis 88,99 Gew.-% der photopolymerisierbaren Zusammensetzung, bestehend aus Poly(ethylenglykol)methacrylat (PEGMA) und Poly(ethylenglykol)diacrylat (PEGDA),
d) 0,01 bis 5 Gew.-% eines Photoinitiators, ausgewählt aus der Gruppe bestehend aus 1-[4-(2-Hydroxyethoxy)-phenyl]-2-hydroxy-2-methyl-1-propanon, Phenyl-bis(2,4,6-trimethylbenzoyl)phosphinoxid und Gemische davon,
wobei das Gewichtsverhältnis PEGOA:PEGMA zwischen 0,5 und 20, 0,5 und 15, 1 und 15, 1 und 10, oder 2 und 5 liegt.

12. Okuläre Zusammensetzung nach einem vorstehenden Anspruch, wobei der therapeutische Wirkstoff Ranibizumab, Bevacizumab, Latanoprost, Dexamethason oder Timololmaleat ist.

13. Verfahren zur Herstellung eines Okularimplantats, das die folgenden Schritte umfasst:
a) Bereitstellen einer okulären Zusammensetzung nach einem der Ansprüche 1 bis 12; und
b) Bestrahlen der okulären Zusammensetzung mit Licht bei einer Wellenlänge zwischen 200 und 550 nm für einen Zeitraum zwischen 1 Sekunde und 60 Minuten, um das Okularimplantat zu bilden.

14. Okularimplantat, das mindestens 0,1 Gew.-% eines therapeutischen Wirkstoffs, 5 bis 95 Gew.-% einer vernetzten Polymermatrix und 0,1 bis 40 Gew.-% eines biologisch abbaubaren Polymers umfasst, das ausgewählt ist aus der Gruppe bestehend aus Lactid/Glycolid-Copolymer (einschließlich Poly(lactid-co-glycolid) (PLGA)), Poly(L-lactid) (PLA), Polyhydroxyalkanoaten, einschließlich Polyhydroxybutyrat, Polyglykolsäure (PGA), Polycaprolacton (PCL), Poly(OL-lactid) (PDL), Poly(D-lactid), Lactid/Caprolacton-Copolymer, Poly-L-lactid-co-caprolacton (PLC) und Mischungen, Copolymeren und Blockcopolymeren davon, **dadurch gekennzeichnet, dass**:
a) die vernetzte Polymermatrix durch Vernetzen einer photopolymerisierbaren Zusammensetzung erhalten wird, die 3 bis 70 Gew.-% einer oder mehrerer Verbindungen der Formel I umfasst: 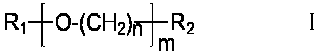
wobei R₁ Wasserstoff oder ein lineares oder verzweigtes C₁-C₃-Alkyl ist; R₂ eine Acrylat- oder Methacrylatgruppe ist; n 2 oder 3 ist und m gleich oder größer als 1 ist, wobei der Gewichtsprozentanteil der einen oder mehreren Verbindungen der Formel I auf das Gesamtgewicht der photopolymerisierbaren Zusammensetzung bezogen ist,
wobei die photopolymerisierbare Zusammensetzung weiter eine oder mehrere disubstituierte Acrylat- oder Methacrylatverbindungen umfasst, die aus der Gruppe ausgewählt sind, bestehend aus Ethylenglykoldiacrylat, Di(ethylenglykol)diacrylat, Poly(ethylenglykol)diacrylat, Ethylenglykoldimethacrylat, Di(ethylenglykol)dimethacrylat, Poly(ethylenglykol)dimethacrylat, Propylenglykoldiacrylat, Di(propylenglykol)diacrylat, Poly(propylenglykol)diacrylat, Propylenglykoldimethacrylat, Di(propylenglykol)dimethacrylat, Poly(propylenglykol)dimethacrylat, 1,6-Hexandioldimethacrylat;
b) der therapeutische Wirkstoff und das biologisch abbaubare Polymer in die Polymermatrix eingebettet sind.

15. Okularimplantat nach Anspruch 14, umfassend oder bestehend aus:
a) 10 bis 50 Gew.-% des therapeutischen Wirkstoffs;
b) 1 bis 20 Gew.-% Poly(lactid-co-glycolid) (PLGA);
c) 30 bis 89 Gew.-% der vernetzten Polymermatrix, erhalten durch Vernetzung der photopolymerisierbaren Zusammensetzung, bestehend aus Poly(ethylenglykol)methacrylat (PEGMA) und Poly(ethylenglykol)diacrylat (PEGDA), wobei das Gewichtsverhältnis PEGOA:PEGMA zwischen 0,5 und 20, 0,5 und 15, 1 und 15, 1 und 10, oder 2 und 5 liegt.

## Revendications

1. Composition oculaire comprenant :
a) au moins 0,1 % en poids d'un agent thérapeutique ;
b) de 5 à 95 % en poids d'une composition photopolymérisable comprenant de 3 à 70 % en poids d'un ou plusieurs composés de formule I : 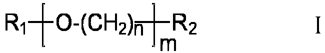
dans laquelle R₁ est de l'hydrogène ou un alkyle en C₁-C₃ linéaire ou ramifié ; R₂ est un groupe acrylate ou méthacrylate ; n vaut 2 ou 3 et m est supérieur ou égal à 1, le pourcentage en poids des un ou plusieurs composés de formule I étant basé sur le poids total de la composition photopolymérisable,
dans laquelle la composition photopolymérisable comprend en outre un ou plusieurs composés d'acrylate ou de méthacrylate disubstitués sélectionnés parmi le groupe consistant en diacrylate d'éthylène glycol, diacrylate de di(éthylène glycol), diacrylate de poly(éthylène glycol), diméthacrylate d'éthylène glycol, diméthacrylate de di(éthylène glycol), diméthacrylate de poly(éthylène glycol), diacrylate de propylène glycol, diacrylate de di(propylène glycol), diacrylate de poly(propylène glycol), diméthacrylate de propylène glycol, diméthacrylate de di(propylène glycol), diméthacrylate de poly(propylène glycol), diméthacrylate de 1,6-hexanediol ;
c) de 0,1 à 40 % en poids d'un polymère biodégradable sélectionné parmi le groupe consistant en copolymère de lactide/glycolide (incluant poly(lactide-co-glycolide) (PLGA)), poly(L-lactide) (PLA), polyhydroxyalcanoates, incluant polyhydroxybutyrate, acide polyglycolique (PGA), polycaprolactone (PCL), poly (DL-lactide) (PDL), poly(O-lactide), copolymère de lactide/caprolactone, poly-L-lactide-co-caprolactone (PLC) et des mélanges, copolymères et copolymères séquencés de ceux-ci, et
d) un photo-initiateur.

2. Composition oculaire selon la revendication 1, dans laquelle le composé de formule I est sélectionné parmi le groupe consistant en acrylate de poly(éthylène glycol), méthacrylate de poly(éthylène glycol), acrylate d'éther méthylique de poly(éthylène glycol), méthacrylate d'éther méthylique de poly(éthylène glycol), acrylate d'éther méthylique d'éthylène glycol, acrylate d'éther éthylique de di(éthylène glycol), méthacrylate d'éther méthylique d'éthylène glycol, méthacrylate d'éther éthylique de di(éthylène glycol) et des mélanges de ceux-ci.

3. Composition oculaire selon la revendication 2, dans laquelle le composé de formule I est du méthacrylate de poly(éthylène glycol) (PEGMA).

4. Composition oculaire selon une quelconque revendication précédente, dans laquelle la composition photopolymérisable comprend de 5 à 60 % en poids d'un ou plusieurs composés de formule I.

5. Composition oculaire selon une quelconque revendication précédente, dans laquelle les un ou plusieurs composés d'acrylate ou de méthacrylate disubstitués sont sélectionnés parmi le groupe consistant en diacrylate de poly(éthylène glycol) et diméthacrylate de poly(éthylène glycol).

6. Composition oculaire selon la revendication 5, dans laquelle les un ou plusieurs composés d'acrylate ou de méthacrylate disubstitués sont du diacrylate de poly(éhylène glycol) (PEGDA).

7. Composition oculaire selon une quelconque revendication précédente, comprenant de 40 à 75 % en poids de la composition photopolymérisable.

8. Composition oculaire selon une quelconque revendication précédente, dans laquelle le polymère biodégradable est un copolymère de lactide/glycolide (incluant poly(L-lactide-co-glycolide) (PLGA)), poly(L-lactide) (PLA), poly(DL-lactide) (PDL) et un copolymère de lactide/caprolactone (PLC).

9. Composition oculaire selon la revendication 8, dans laquelle le polymère biodégradable est du poly(lactide-co-glycolide (PLGA).

10. Composition oculaire selon une quelconque revendication précédente, dans laquelle l'agent thérapeutique est présent en une quantité entre 20 et 70 % en poids.

11. Composition oculaire selon une quelconque revendication précédente, comprenant
a) de 10 à 50 % en poids de l'agent thérapeutique
b) de 1 à 20 % en poids de poly(lactide-co-glycolide) (PLGA)
c) de 30 à 88,99 % en poids de la composition photopolymérisable consistant en méthacrylate de poly(éthylène glycol) (PEGMA) et diacrylate de poly(éthylène glycol) (PEGDA)
d) de 0,01 à 5 % en poids d'un photo-initiateur sélectionné parmi le groupe consistant en 1-[4-(2-hydroxyéthoxy)-phényl]-2-hydroxy-2-méthyl-1-propanone, phényl-bis(2,4,6-triméthylbenzoyl)-phosphineoxyde et des mélanges de ceux-ci
dans laquelle le rapport pondéral PEGDA:PEGMA est entre 0,5 et 20, 0,5 et 15, 1 et 15, 1 et 10, ou 2 et 5.

12. Composition oculaire selon une quelconque revendication précédente, dans laquelle l'agent thérapeutique est ranibizumab, bevacizumab, latanoprost, dexaméthasone ou maléate de timolol.

13. Procédé de fabrication d'un implant oculaire, comprenant les étapes consistant à :
a) fournir une composition oculaire selon l'une quelconque des revendications 1 à 12 ; et
b) exposer la composition oculaire à de la lumière à une longueur d'onde entre 200 et 550 nm pendant une période de temps entre 1 seconde et 60 minutes pour former l'implant oculaire.

14. Implant oculaire comprenant au moins 0,1 % en poids d'un agent thérapeutique, de 5 à 95 % en poids d'une matrice polymère réticulée et de 0,1 à 40 % en poids d'un polymère biodégradable sélectionné parmi le groupe consistant en copolymère de lactide/glycolide (incluant poly(lactide-co-glycolide) (PLGA)), poly(L-lactide) (PLA), polyhydroxyalcanoates, incluant polyhydroxybutyrate, acide polyglycolique (PGA), polycaprolactone (PCL), poly(DL-lactide) (PDL), poly(O-lactide), copolymère de lactide/caprolactone, poly-L-lactide-co-caprolactone (PLC) et des mélanges, copolymères, et copolymères séquencés de ceux-ci, **caractérisé en ce que** :
a) la matrice polymère réticulée est obtenue en réticulant une composition photopolymérisable comprenant de 3 à 70 % en poids d'un ou plusieurs composés de formule I : 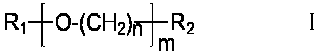
dans laquelle R₁ est de l'hydrogène ou un alkyle en C₁-C₃ linéaire ou ramifié ; R₂ est un groupe acrylate ou méthacrylate ; n vaut 2 ou 3 et m est supérieur ou égal à 1, le pourcentage en poids des un ou plusieurs composés de formule I étant basé sur le poids total de la composition photopolymérisable,
dans laquelle la composition photopolymérisable comprend en outre un ou plusieurs composés d'acrylate ou de méthacrylate disubstitués sélectionnés parmi le groupe consistant en diacrylate d'éthylène glycol, diacrylate de di(éthylène glycol), diacrylate de poly(éthylène glycol), diméthacrylate d'éthylène glycol, diméthacrylate de di(éthylène glycol), diméthacrylate de poly(éthylène glycol), diacrylate de propylène glycol, diacrylate de di(propylène glycol), diacrylate de poly(propylène glycol), diméthacrylate de propylène glycol, diméthacrylate de di(propylène glycol), diméthacrylate de poly(propylène glycol), diméthacrylate de 1,6-hexanediol ;
b) l'agent thérapeutique et le polymère biodégradable sont incorporés dans la matrice polymère.

15. Implant oculaire selon la revendication 14, comprenant ou consistant en :
a) de 10 à 50 % en poids de l'agent thérapeutique ;
b) de 1 à 20 % en poids de poly(lactide-co-glycolide) (PLGA) ;
c) de 30 à 89 % en poids de la matrice polymère réticulée obtenue en réticulant la composition photopolymérisable consistant en méthacrylate de poly(éthylène glycol) (PEGMA) et diacrylate de poly(éthylène glycol) (PEGDA), dans lequel le rapport pondéral PEGDA:PEGMA est entre 0,5 et 20, 0,5 et 15, 1 et 15, 1 et 10, ou 2 et 5.
